(19) **Europäisches Patentamt / European Patent Office / Office européen des brevets**

(11) **EP 2 975 056 A1**

(12) **EUROPEAN PATENT APPLICATION**

(43) Date of publication:
**20.01.2016 Bulletin 2016/03**

(51) Int Cl.:
***C07K 16/28*** (2006.01)

(21) Application number: **14177547.8**

(22) Date of filing: **17.07.2014**

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR**<br>Designated Extension States:<br>**BA ME**<br><br>(71) Applicant: **NOVO NORDISK A/S**<br>**2880 Bagsværd (DK)** | (72) Inventor: **The designation of the inventor has not yet been filed**<br><br>(74) Representative: **Griffin, Philippa Jane et al**<br>**Mathys & Squire LLP**<br>**The Shard**<br>**32 London Bridge Street**<br>**London SE1 9SG (GB)** |

(54) **Site directed mutagenesis of TREM-1 antibodies for decreasing viscosity**

(57) Antibodies that are capable of specifically binding and preventing the activation of TREM-1, a protein expressed on monocytes, macrophages and neutrophils with both good affinity and low viscosity at clinically relevant concentrations are described. Such antibodies find utility in the treatment of individuals with an inflammatory disease, such as rheumatoid arthritis and inflammatory bowel disease.

EP 2 975 056 A1

SEQUENCE LISTING

<110>   Novo Nordisk A/S

<120>   SITE DIRECTED MUTAGENESIS OF TREM-1 ANTIBODIES

<130>   140049

<160>   17

<170>   PatentIn version 3.5

<210>   1
<211>   234
<212>   PRT
<213>   Homo Sapiens

<400>   1

Met Arg Lys Thr Arg Leu Trp Gly Leu Leu Trp Met Leu Phe Val Ser
1               5                   10                  15

Glu Leu Arg Ala Ala Thr Lys Leu Thr Glu Glu Lys Tyr Glu Leu Lys
            20                  25                  30

Glu Gly Gln Thr Leu Asp Val Lys Cys Asp Tyr Thr Leu Glu Lys Phe
            35                  40                  45

Ala Ser Ser Gln Lys Ala Trp Gln Ile Ile Arg Asp Gly Glu Met Pro
        50                  55                  60

Lys Thr Leu Ala Cys Thr Glu Arg Pro Ser Lys Asn Ser His Pro Val
65                  70                  75                  80

Gln Val Gly Arg Ile Ile Leu Glu Asp Tyr His Asp His Gly Leu Leu
                85                  90                  95

Arg Val Arg Met Val Asn Leu Gln Val Glu Asp Ser Gly Leu Tyr Gln
            100                 105                 110

Cys Val Ile Tyr Gln Pro Pro Lys Glu Pro His Met Leu Phe Asp Arg
            115                 120                 125

Ile Arg Leu Val Val Thr Lys Gly Phe Ser Gly Thr Pro Gly Ser Asn
        130                 135                 140

Glu Asn Ser Thr Gln Asn Val Tyr Lys Ile Pro Pro Thr Thr Thr Lys
145                 150                 155                 160

Ala Leu Cys Pro Leu Tyr Thr Ser Pro Arg Thr Val Thr Gln Ala Pro
                165                 170                 175

```
Pro Lys Ser Thr Ala Asp Val Ser Thr Pro Asp Ser Glu Ile Asn Leu
        180                 185                 190


Thr Asn Val Thr Asp Ile Ile Arg Val Pro Val Phe Asn Ile Val Ile
        195                 200                 205


Leu Leu Ala Gly Gly Phe Leu Ser Lys Ser Leu Val Phe Ser Val Leu
        210                 215                 220


Phe Ala Val Thr Leu Arg Ser Phe Val Pro
225                 230
```

```
<210>  2
<211>  448
<212>  PRT
<213>  Artificial Sequence

<220>
<223>  heavy chain of a humanised TREM-1 antibody


<400>  4

Glu Val Gln Leu Val Glu Ser Gly Gly Gly Leu Val Gln Pro Gly Gly
1                 5                 10                 15


Ser Leu Lys Leu Ser Cys Ala Ala Ser Gly Phe Thr Phe Ser Thr Tyr
        20                 25                 30


Ala Met His Trp Val Arg Gln Ala Ser Gly Lys Gly Leu Glu Trp Val
        35                 40                 45


Gly Arg Ile Arg Thr Lys Ser Ser Asn Tyr Ala Thr Tyr Tyr Ala Ala
        50                 55                 60


Ser Val Lys Gly Arg Phe Thr Ile Ser Arg Asp Asp Ser Lys Asn Thr
65                 70                 75                 80


Ala Tyr Leu Gln Met Asn Ser Leu Lys Thr Glu Asp Thr Ala Val Tyr
                85                 90                 95


Tyr Cys Thr Arg Asp Met Gly Ile Arg Arg Gln Phe Ala Tyr Trp Gly
                100                 105                 110


Gln Gly Thr Leu Val Thr Val Ser Ser Ala Ser Thr Lys Gly Pro Ser
        115                 120                 125
```

```
Val Phe Pro Leu Ala Pro Cys Ser Arg Ser Thr Ser Glu Ser Thr Ala
    130             135             140

Ala Leu Gly Cys Leu Val Lys Asp Tyr Phe Pro Glu Pro Val Thr Val
    145             150             155             160

Ser Trp Asn Ser Gly Ala Leu Thr Ser Gly Val His Thr Phe Pro Ala
                165             170             175

Val Leu Gln Ser Ser Gly Leu Tyr Ser Leu Ser Ser Val Val Thr Val
            180             185             190

Pro Ser Ser Ser Leu Gly Thr Lys Thr Tyr Thr Cys Asn Val Asp His
        195             200             205

Lys Pro Ser Asn Thr Lys Val Asp Lys Arg Val Glu Ser Lys Tyr Gly
    210             215             220

Pro Pro Cys Pro Pro Cys Pro Ala Pro Glu Phe Leu Gly Gly Pro Ser
225             230             235             240

Val Phe Leu Phe Pro Pro Lys Pro Lys Asp Thr Leu Met Ile Ser Arg
            245             250             255

Thr Pro Glu Val Thr Cys Val Val Val Asp Val Ser Gln Glu Asp Pro
            260             265             270

Glu Val Gln Phe Asn Trp Tyr Val Asp Gly Val Glu Val His Asn Ala
            275             280             285

Lys Thr Lys Pro Arg Glu Glu Gln Phe Asn Ser Thr Tyr Arg Val Val
    290             295             300

Ser Val Leu Thr Val Leu His Gln Asp Trp Leu Asn Gly Lys Glu Tyr
305             310             315             320

Lys Cys Lys Val Ser Asn Lys Gly Leu Pro Ser Ser Ile Glu Lys Thr
            325             330             335

Ile Ser Lys Ala Lys Gly Gln Pro Arg Glu Pro Gln Val Tyr Thr Leu
            340             345             350

Pro Pro Ser Gln Glu Glu Met Thr Lys Asn Gln Val Ser Leu Thr Cys
        355             360             365

Leu Val Lys Gly Phe Tyr Pro Ser Asp Ile Ala Val Glu Trp Glu Ser
    370             375             380
```

```
Asn Gly Gln Pro Glu Asn Asn Tyr Lys Thr Thr Pro Pro Val Leu Asp
385             390             395             400
```

```
Ser Asp Gly Ser Phe Phe Leu Tyr Ser Arg Leu Thr Val Asp Lys Ser
            405             410             415
```

```
Arg Trp Gln Glu Gly Asn Val Phe Ser Cys Ser Val Met His Glu Ala
        420             425             430
```

```
Leu His Asn His Tyr Thr Gln Lys Ser Leu Ser Leu Ser Leu Gly Lys
        435             440             445
```

```
<210>  3
<211>  218
<212>  PRT
<213>  Artificial Sequence

<220>
<223>  light chain of a humanised TREM-1 antibody


<220>
<221>  CHAIN
<222>  (1)..(218)
<223>  light chain of a humanised TREM-1 antibody
```

```
Asp Ile Val Leu Thr Gln Ser Pro Asp Ser Leu Ala Val Ser Leu Gly
1               5               10              15
```

```
Glu Arg Ala Thr Ile Asn Cys Arg Ala Ser Glu Ser Val Asp Thr Phe
        20              25              30
```

```
Asp Tyr Ser Phe Leu His Trp Tyr Gln Gln Lys Pro Gly Gln Pro Pro
        35              40              45
```

```
Lys Leu Leu Ile Tyr Arg Ala Ser Asn Leu Glu Ser Gly Val Pro Asp
    50              55              60
```

```
Arg Phe Ser Gly Ser Gly Ser Gly Thr Asp Phe Thr Leu Thr Ile Ser
65              70              75              80
```

```
Ser Leu Gln Ala Glu Asp Val Ala Val Tyr Tyr Cys Gln Gln Ser Asn
            85              90              95
```

```
Glu Asp Pro Tyr Thr Phe Gly Gln Gly Thr Lys Leu Glu Ile Lys Arg
            100             105             110
```

```
Thr Val Ala Ala Pro Ser Val Phe Ile Phe Pro Pro Ser Asp Glu Gln
        115             120             125
```

```
Leu Lys Ser Gly Thr Ala Ser Val Val Cys Leu Leu Asn Asn Phe Tyr
    130                 135             140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
            195                 200                 205

Val Thr Lys Ser Phe Asn Arg Gly Glu Cys
    210                 215
```

```
<210>  4
<211>  218
<212>  PRT
<213>  Artificial Sequence

<220>
<223>  light chain of a humanised TREM-1 antibody
```

```
Asp Ile Val Leu Thr Gln Ser Pro Asp Ser Leu Ala Val Ser Leu Gly
1                   5                   10                  15

Glu Arg Ala Thr Ile Asn Cys Arg Ala Ser Gln Ser Val Asp Thr Phe
                20                  25                  30

Asp Tyr Ser Phe Leu His Trp Tyr Gln Gln Lys Pro Gly Gln Pro Pro
            35                  40                  45

Lys Leu Leu Ile Tyr Arg Ala Ser Asn Leu Glu Ser Gly Val Pro Asp
    50                  55                  60

Arg Phe Ser Gly Ser Gly Ser Gly Thr Asp Phe Thr Leu Thr Ile Ser
65                  70                  75                  80

Ser Leu Gln Ala Glu Asp Val Ala Val Tyr Tyr Cys Gln Gln Ser Asn
                85                  90                  95

Ser Asp Pro Tyr Thr Phe Gly Gln Gly Thr Lys Leu Glu Ile Lys Arg
            100                 105                 110
```

```
Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
        130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
                180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215

<210>  5
<211>  218
<212>  PRT
<213>  Artificial Sequence


Asp Ile Val Leu Thr Gln Ser Pro Asp Ser Leu Ala Val Ser Leu Gly
1                   5                   10                  15

Glu Arg Ala Thr Ile Asn Cys Arg Ala Ser Gln Ser Val Asp Thr Phe
        20                  25                  30

Asp Tyr Ser Phe Leu His Trp Tyr Gln Gln Lys Pro Gly Gln Pro Pro
        35                  40                  45

Lys Leu Leu Ile Tyr Arg Ala Ser Asn Leu Glu Ser Gly Val Pro Asp
    50                  55                  60

Arg Phe Ser Gly Ser Gly Ser Gly Thr Asp Phe Thr Leu Thr Ile Ser
65                  70                  75                  80

Ser Leu Gln Ala Glu Asp Val Ala Val Tyr Tyr Cys Gln Gln Ser Asn
                85                  90                  95

Gln Asp Pro Tyr Thr Phe Gly Gln Gly Thr Lys Leu Glu Ile Lys Arg
```

                    100                    105                    110

Thr Val Ala Ala Pro Ser Val Phe Ile Phe Pro Pro Ser Asp Glu Gln
        115                    120                    125

Leu Lys Ser Gly Thr Ala Ser Val Val Cys Leu Leu Asn Asn Phe Tyr
        130                    135                    140

Pro Arg Glu Ala Lys Val Gln Trp Lys Val Asp Asn Ala Leu Gln Ser
145                    150                    155                    160

Gly Asn Ser Gln Glu Ser Val Thr Glu Gln Asp Ser Lys Asp Ser Thr
                165                    170                    175

Tyr Ser Leu Ser Ser Thr Leu Thr Leu Ser Lys Ala Asp Tyr Glu Lys
            180                    185                    190

His Lys Val Tyr Ala Cys Glu Val Thr His Gln Gly Leu Ser Ser Pro
        195                    200                    205

Val Thr Lys Ser Phe Asn Arg Gly Glu Cys
        210                    215

<210> 6
<211> 218
<212> PRT
<213> Artificial Sequence

<220>
<223> light chain of a humanised TREM-1 antibody


Asp Ile Val Leu Thr Gln Ser Pro Asp Ser Leu Ala Val Ser Leu Gly
1                    5                    10                    15

Glu Arg Ala Thr Ile Asn Cys Arg Ala Ser Glu Ser Val Asp Thr Phe
            20                    25                    30

Asp Tyr Ser Phe Leu His Trp Tyr Gln Gln Lys Pro Gly Gln Pro Pro
        35                    40                    45

Lys Leu Leu Ile Tyr Arg Ala Ser Asn Leu Glu Ser Gly Val Pro Asp
        50                    55                    60

Arg Phe Ser Gly Ser Gly Ser Gly Thr Asp Phe Thr Leu Thr Ile Ser
65                    70                    75                    80

Ser Leu Gln Ala Glu Asp Val Ala Val Tyr Tyr Cys Gln Gln Ser Asn

EP 2 975 056 A1

```
                    85                    90                        95


        Ser Asp Pro Tyr Thr Phe Gly Gln Gly Thr Lys Leu Glu Ile Lys Arg
                    100                 105                 110


        Thr Val Ala Ala Pro Ser Val Phe Ile Phe Pro Pro Ser Asp Glu Gln
                    115                 120                 125


        Leu Lys Ser Gly Thr Ala Ser Val Val Cys Leu Leu Asn Asn Phe Tyr
                    130                 135                 140


        Pro Arg Glu Ala Lys Val Gln Trp Lys Val Asp Asn Ala Leu Gln Ser
        145                 150                 155                 160


        Gly Asn Ser Gln Glu Ser Val Thr Glu Gln Asp Ser Lys Asp Ser Thr
                    165                 170                 175


        Tyr Ser Leu Ser Ser Thr Leu Thr Leu Ser Lys Ala Asp Tyr Glu Lys
                    180                 185                 190


        His Lys Val Tyr Ala Cys Glu Val Thr His Gln Gly Leu Ser Ser Pro
                    195                 200                 205


        Val Thr Lys Ser Phe Asn Arg Gly Glu Cys
                    210                 215


        <210>  7
        <211>  218
        <212>  PRT
        <213>  Artificial Sequence

        <220>
        <223>  light chain of a humanised TREM-1 antibody


        Asp Ile Val Leu Thr Gln Ser Pro Asp Ser Leu Ala Val Ser Leu Gly
        1                   5                   10                  15


        Glu Arg Ala Thr Ile Asn Cys Arg Ala Ser Glu Ser Val Asp Thr Phe
                    20                  25                  30


        Asp Tyr Ser Phe Leu His Trp Tyr Gln Gln Lys Pro Gly Gln Pro Pro
                    35                  40                  45


        Lys Leu Leu Ile Tyr Arg Ala Ser Asn Leu Glu Ser Gly Val Pro Asp
                    50                  55                  60


        Arg Phe Ser Gly Ser Gly Ser Gly Thr Asp Phe Thr Leu Thr Ile Ser
        65                  70                  75                  80
```

27

```
Ser Leu Gln Ala Glu Asp Val Ala Val Tyr Tyr Cys Gln Gln Ser Asn
                85                      90                  95

Gln Asp Pro Tyr Thr Phe Gly Gln Gly Thr Lys Leu Glu Ile Lys Arg
                100                     105                 110

Thr Val Ala Ala Pro Ser Val Phe Ile Phe Pro Pro Ser Asp Glu Gln
                115                     120                 125

Leu Lys Ser Gly Thr Ala Ser Val Val Cys Leu Leu Asn Asn Phe Tyr
        130                     135                 140

Pro Arg Glu Ala Lys Val Gln Trp Lys Val Asp Asn Ala Leu Gln Ser
145                     150                     155                 160

Gly Asn Ser Gln Glu Ser Val Thr Glu Gln Asp Ser Lys Asp Ser Thr
                165                     170                 175

Tyr Ser Leu Ser Ser Thr Leu Thr Leu Ser Lys Ala Asp Tyr Glu Lys
                180                     185                 190

His Lys Val Tyr Ala Cys Glu Val Thr His Gln Gly Leu Ser Ser Pro
        195                     200                 205

Val Thr Lys Ser Phe Asn Arg Gly Glu Cys
    210                     215
```

```
<210>  8
<211>  218
<212>  PRT
<213>  Artificial Sequence

<220>
<223>  light chain of a humanised TREM-1 antibody
```

```
Asp Ile Val Leu Thr Gln Ser Pro Asp Ser Leu Ala Val Ser Leu Gly
1               5               10                  15

Glu Arg Ala Thr Ile Asn Cys Arg Ala Ser Gln Ser Val Asp Thr Phe
                20                  25                  30

Asp Tyr Ser Phe Leu His Trp Tyr Gln Gln Lys Pro Gly Gln Pro Pro
        35                  40                  45

Lys Leu Leu Ile Tyr Arg Ala Ser Asn Leu Glu Ser Gly Val Pro Asp
        50                  55                  60
```

Arg Phe Ser Gly Ser Gly Ser Gly Thr Asp Phe Thr Leu Thr Ile Ser
65              70              75              80

Ser Leu Gln Ala Glu Asp Val Ala Val Tyr Tyr Cys Gln Gln Ser Asn
                85              90              95

Glu Asp Pro Tyr Thr Phe Gly Gln Gly Thr Lys Leu Glu Ile Lys Arg
            100             105             110

Thr Val Ala Ala Pro Ser Val Phe Ile Phe Pro Pro Ser Asp Glu Gln
        115             120             125

Leu Lys Ser Gly Thr Ala Ser Val Val Cys Leu Leu Asn Asn Phe Tyr
    130             135             140

Pro Arg Glu Ala Lys Val Gln Trp Lys Val Asp Asn Ala Leu Gln Ser
145             150             155             160

Gly Asn Ser Gln Glu Ser Val Thr Glu Gln Asp Ser Lys Asp Ser Thr
            165             170             175

Tyr Ser Leu Ser Ser Thr Leu Thr Leu Ser Lys Ala Asp Tyr Glu Lys
        180             185             190

His Lys Val Tyr Ala Cys Glu Val Thr His Gln Gly Leu Ser Ser Pro
        195             200             205

Val Thr Lys Ser Phe Asn Arg Gly Glu Cys
    210             215

```
<210>  9
<211>  218
<212>  PRT
<213>  Artificial Sequence

<220>
<223>  light chain of a humanised TREM-1 antibody
```

Asp Ile Val Leu Thr Gln Ser Pro Asp Ser Leu Ala Val Ser Leu Gly
1           5               10              15

Glu Arg Ala Thr Ile Asn Cys Arg Ala Ser Glu Ser Val Asp Thr Ala
            20              25              30

Asp Tyr Ser Phe Leu His Trp Tyr Gln Gln Lys Pro Gly Gln Pro Pro
        35              40              45

```
Lys Leu Leu Ile Tyr Arg Ala Ser Asn Leu Glu Ser Gly Val Pro Asp
    50              55              60

Arg Phe Ser Gly Ser Gly Ser Gly Thr Asp Phe Thr Leu Thr Ile Ser
65              70              75              80

Ser Leu Gln Ala Glu Asp Val Ala Val Tyr Tyr Cys Gln Gln Ser Asn
            85              90              95

Glu Asp Pro Tyr Thr Phe Gly Gln Gly Thr Lys Leu Glu Ile Lys Arg
        100             105             110

Thr Val Ala Ala Pro Ser Val Phe Ile Phe Pro Pro Ser Asp Glu Gln
        115             120             125

Leu Lys Ser Gly Thr Ala Ser Val Val Cys Leu Leu Asn Asn Phe Tyr
    130             135             140

Pro Arg Glu Ala Lys Val Gln Trp Lys Val Asp Asn Ala Leu Gln Ser
145             150             155             160

Gly Asn Ser Gln Glu Ser Val Thr Glu Gln Asp Ser Lys Asp Ser Thr
            165             170             175

Tyr Ser Leu Ser Ser Thr Leu Thr Leu Ser Lys Ala Asp Tyr Glu Lys
        180             185             190

His Lys Val Tyr Ala Cys Glu Val Thr His Gln Gly Leu Ser Ser Pro
    195             200             205

Val Thr Lys Ser Phe Asn Arg Gly Glu Cys
    210             215
```

```
<210>  10
<211>  218
<212>  PRT
<213>  Artificial Sequence

<220>
<223>  light chain of a humanised TREM-1 antibody
```

```
Asp Ile Val Leu Thr Gln Ser Pro Asp Ser Leu Ala Val Ser Leu Gly
1               5               10              15

Glu Arg Ala Thr Ile Asn Cys Arg Ala Ser Glu Ser Val Asp Thr Ser
            20              25              30
```

```
Asp Tyr Ser Phe Leu His Trp Tyr Gln Gln Lys Pro Gly Gln Pro Pro
        35              40                  45


Lys Leu Leu Ile Tyr Arg Ala Ser Asn Leu Glu Ser Gly Val Pro Asp
        50              55                  60


Arg Phe Ser Gly Ser Gly Ser Gly Thr Asp Phe Thr Leu Thr Ile Ser
65              70              75                  80


Ser Leu Gln Ala Glu Asp Val Ala Val Tyr Tyr Cys Gln Gln Ser Asn
                85              90                  95


Glu Asp Pro Tyr Thr Phe Gly Gln Gly Thr Lys Leu Glu Ile Lys Arg
            100             105             110


Thr Val Ala Ala Pro Ser Val Phe Ile Phe Pro Pro Ser Asp Glu Gln
        115             120             125


Leu Lys Ser Gly Thr Ala Ser Val Val Cys Leu Leu Asn Asn Phe Tyr
    130             135             140


Pro Arg Glu Ala Lys Val Gln Trp Lys Val Asp Asn Ala Leu Gln Ser
145             150             155             160


Gly Asn Ser Gln Glu Ser Val Thr Glu Gln Asp Ser Lys Asp Ser Thr
            165             170             175


Tyr Ser Leu Ser Ser Thr Leu Thr Leu Ser Lys Ala Asp Tyr Glu Lys
        180             185             190


His Lys Val Tyr Ala Cys Glu Val Thr His Gln Gly Leu Ser Ser Pro
    195             200             205


Val Thr Lys Ser Phe Asn Arg Gly Glu Cys
    210             215
```

```
<210>  11
<211>  448
<212>  PRT
<213>  Artificial Sequence

<220>
<223>  heavy chain of a humanised TREM-1 antibody
```

```
Glu Val Gln Leu Val Glu Ser Gly Gly Gly Leu Val Gln Pro Gly Gly
1               5                   10                  15
```

Ser Leu Lys Leu Ser Cys Ala Ala Ser Gly Phe Thr Phe Ser Thr Tyr
            20                  25                  30

Ala Met His Trp Val Arg Gln Ala Ser Gly Lys Gly Leu Glu Trp Val
            35                  40                  45

Gly Arg Ile Arg Thr Lys Ser Ser Asn Tyr Tyr Thr Tyr Tyr Ala Ala
            50                  55                  60

Ser Val Lys Gly Arg Phe Thr Ile Ser Arg Asp Asp Ser Lys Asn Thr
65                  70                  75                  80

Ala Tyr Leu Gln Met Asn Ser Leu Lys Thr Glu Asp Thr Ala Val Tyr
                85                  90                  95

Tyr Cys Thr Arg Asp Met Gly Ile Arg Arg Gln Phe Ala Tyr Trp Gly
            100                 105                 110

Gln Gly Thr Leu Val Thr Val Ser Ser Ala Ser Thr Lys Gly Pro Ser
            115                 120                 125

Val Phe Pro Leu Ala Pro Cys Ser Arg Ser Thr Ser Glu Ser Thr Ala
            130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Lys Thr Tyr Thr Cys Asn Val Asp His
            195                 200                 205

Lys Pro Ser Asn Thr Lys Val Asp Lys Arg Val Glu Ser Lys Tyr Gly
            210                 215                 220

Pro Pro Cys Pro Pro Cys Pro Ala Pro Glu Phe Leu Gly Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser Gln Glu Asp Pro
                260                 265                 270

```
Glu Val Gln Phe Asn Trp Tyr Val Asp Gly Val Glu Val His Asn Ala
        275                 280                 285

Lys Thr Lys Pro Arg Glu Glu Gln Phe Asn Ser Thr Tyr Arg Val Val
        290                 295                 300

Ser Val Leu Thr Val Leu His Gln Asp Trp Leu Asn Gly Lys Glu Tyr
305                 310                 315                 320

Lys Cys Lys Val Ser Asn Lys Gly Leu Pro Ser Ser Ile Glu Lys Thr
                325                 330                 335

Ile Ser Lys Ala Lys Gly Gln Pro Arg Glu Pro Gln Val Tyr Thr Leu
        340                 345                 350

Pro Pro Ser Gln Glu Glu Met Thr Lys Asn Gln Val Ser Leu Thr Cys
        355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Arg Leu Thr Val Asp Lys Ser
                405                 410                 415

Arg Trp Gln Glu Gly Asn Val Phe Ser Cys Ser Val Met His Glu Ala
        420                 425                 430

Leu His Asn His Tyr Thr Gln Lys Ser Leu Ser Leu Ser Leu Gly Lys
        435                 440                 445


<210>  12
<211>  448
<212>  PRT
<213>  Artificial Sequence

<220>
<223>  heavy chain of a humanised TREM-1 antibody


Glu Val Gln Leu Val Glu Ser Gly Gly Gly Leu Val Gln Pro Gly Gly
1               5               10                  15

Ser Leu Lys Leu Ser Cys Ala Ala Ser Gly Phe Thr Phe Ser Thr Tyr
        20                  25                  30
```

Ala Met His Trp Val Arg Gln Ala Ser Gly Lys Gly Leu Glu Trp Val
        35                  40                  45

Gly Arg Ile Arg Thr Lys Ser Ser Ser Tyr Ala Thr Tyr Tyr Ala Ala
        50                  55                  60

Ser Val Lys Gly Arg Phe Thr Ile Ser Arg Asp Asp Ser Lys Asn Thr
65                  70                  75                  80

Ala Tyr Leu Gln Met Asn Ser Leu Lys Thr Glu Asp Thr Ala Val Tyr
                85                  90                  95

Tyr Cys Thr Arg Asp Met Gly Ile Arg Arg Gln Phe Ala Tyr Trp Gly
            100                 105                 110

Gln Gly Thr Leu Val Thr Val Ser Ser Ala Ser Thr Lys Gly Pro Ser
            115                 120                 125

Val Phe Pro Leu Ala Pro Cys Ser Arg Ser Thr Ser Glu Ser Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Lys Thr Tyr Thr Cys Asn Val Asp His
        195                 200                 205

Lys Pro Ser Asn Thr Lys Val Asp Lys Arg Val Glu Ser Lys Tyr Gly
    210                 215                 220

Pro Pro Cys Pro Pro Cys Pro Ala Pro Glu Phe Leu Gly Gly Pro Ser
225                 230                 235                 240

Val Phe Leu Phe Pro Pro Lys Pro Lys Asp Thr Leu Met Ile Ser Arg
            245                 250                 255

Thr Pro Glu Val Thr Cys Val Val Val Asp Val Ser Gln Glu Asp Pro
            260                 265                 270

Glu Val Gln Phe Asn Trp Tyr Val Asp Gly Val Glu Val His Asn Ala

275                    280                    285

Lys Thr Lys Pro Arg Glu Glu Gln Phe Asn Ser Thr Tyr Arg Val Val
    290                   295                   300

Ser Val Leu Thr Val Leu His Gln Asp Trp Leu Asn Gly Lys Glu Tyr
305                   310                   315                   320

Lys Cys Lys Val Ser Asn Lys Gly Leu Pro Ser Ser Ile Glu Lys Thr
                325                   330                   335

Ile Ser Lys Ala Lys Gly Gln Pro Arg Glu Pro Gln Val Tyr Thr Leu
                340                   345                   350

Pro Pro Ser Gln Glu Glu Met Thr Lys Asn Gln Val Ser Leu Thr Cys
                355                   360                   365

Leu Val Lys Gly Phe Tyr Pro Ser Asp Ile Ala Val Glu Trp Glu Ser
    370                   375                   380

Asn Gly Gln Pro Glu Asn Asn Tyr Lys Thr Thr Pro Pro Val Leu Asp
385                   390                   395                   400

Ser Asp Gly Ser Phe Phe Leu Tyr Ser Arg Leu Thr Val Asp Lys Ser
                405                   410                   415

Arg Trp Gln Glu Gly Asn Val Phe Ser Cys Ser Val Met His Glu Ala
                420                   425                   430

Leu His Asn His Tyr Thr Gln Lys Ser Leu Ser Leu Ser Leu Gly Lys
                435                   440                   445

<210> 13
<211> 448
<212> PRT
<213> Artificial Sequence

<220>
<223> heavy chain of a humanised TREM-1 antibody

Glu Val Gln Leu Val Glu Ser Gly Gly Gly Leu Val Gln Pro Gly Gly
1                   5                   10                   15

Ser Leu Lys Leu Ser Cys Ala Ala Ser Gly Phe Thr Phe Ser Thr Tyr
                20                   25                   30

```
Ala Met His Trp Val Arg Gln Ala Ser Gly Lys Gly Leu Glu Trp Val
        35                  40                  45

Gly Arg Ile Arg Thr Lys Ser Ser Ser Tyr Tyr Thr Tyr Tyr Ala Ala
        50                  55                  60

Ser Val Lys Gly Arg Phe Thr Ile Ser Arg Asp Asp Ser Lys Asn Thr
65                  70                  75                  80

Ala Tyr Leu Gln Met Asn Ser Leu Lys Thr Glu Asp Thr Ala Val Tyr
                85                  90                  95

Tyr Cys Thr Arg Asp Met Gly Ile Arg Arg Gln Phe Ala Tyr Trp Gly
            100                 105                 110

Gln Gly Thr Leu Val Thr Val Ser Ser Ala Ser Thr Lys Gly Pro Ser
            115                 120                 125

Val Phe Pro Leu Ala Pro Cys Ser Arg Ser Thr Ser Glu Ser Thr Ala
        130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
                180                 185                 190

Pro Ser Ser Ser Leu Gly Thr Lys Thr Tyr Thr Cys Asn Val Asp His
        195                 200                 205

Lys Pro Ser Asn Thr Lys Val Asp Lys Arg Val Glu Ser Lys Tyr Gly
        210                 215                 220

Pro Pro Cys Pro Pro Cys Pro Ala Pro Glu Phe Leu Gly Gly Pro Ser
225                 230                 235                 240

Val Phe Leu Phe Pro Pro Lys Pro Lys Asp Thr Leu Met Ile Ser Arg
            245                 250                 255

Thr Pro Glu Val Thr Cys Val Val Val Asp Val Ser Gln Glu Asp Pro
            260                 265                 270

Glu Val Gln Phe Asn Trp Tyr Val Asp Gly Val Glu Val His Asn Ala
        275                 280                 285
```

Lys Thr Lys Pro Arg Glu Glu Gln Phe Asn Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Gly Leu Pro Ser Ser Ile Glu Lys Thr
                325                 330                 335

Ile Ser Lys Ala Lys Gly Gln Pro Arg Glu Pro Gln Val Tyr Thr Leu
            340                 345                 350

Pro Pro Ser Gln Glu Glu Met Thr Lys Asn Gln Val Ser Leu Thr Cys
            355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Arg Leu Thr Val Asp Lys Ser
                405                 410                 415

Arg Trp Gln Glu Gly Asn Val Phe Ser Cys Ser Val Met His Glu Ala
            420                 425                 430

Leu His Asn His Tyr Thr Gln Lys Ser Leu Ser Leu Ser Leu Gly Lys
            435                 440                 445

<210> 14
<211> 448
<212> PRT
<213> Artificial Sequence

<220>
<223> light chain of a humanised TREM-1 antibody

Asp Ile Val Leu Thr Gln Ser Pro Asp Ser Leu Ala Val Ser Leu Gly
1                 5                 10                 15

Glu Arg Ala Thr Ile Asn Cys Arg Ala Ser Gln Ser Val Asp Thr Ala
            20                 25                 30

Asp Tyr Ser Phe Leu His Trp Tyr Gln Gln Lys Pro Gly Gln Pro Pro
        35                 40                 45

Lys Leu Leu Ile Tyr Arg Ala Ser Asn Leu Glu Ser Gly Val Pro Asp

50 55 60

```
Arg Phe Ser Gly Ser Gly Ser Gly Thr Asp Phe Thr Leu Thr Ile Ser
65              70              75              80

Ser Leu Gln Ala Glu Asp Val Ala Val Tyr Tyr Cys Gln Gln Ser Asn
            85              90              95

Gln Asp Pro Tyr Thr Phe Gly Gln Gly Thr Lys Leu Glu Ile Lys Arg
        100             105             110

Thr Val Ala Ala Pro Ser Val Phe Ile Phe Pro Pro Ser Asp Glu Gln
    115             120             125

Leu Lys Ser Gly Thr Ala Ser Val Val Cys Leu Leu Asn Asn Phe Tyr
    130             135             140

Pro Arg Glu Ala Lys Val Gln Trp Lys Val Asp Asn Ala Leu Gln Ser
145             150             155             160

Gly Asn Ser Gln Glu Ser Val Thr Glu Gln Asp Ser Lys Asp Ser Thr
            165             170             175

Tyr Ser Leu Ser Ser Thr Leu Thr Leu Ser Lys Ala Asp Tyr Glu Lys
            180             185             190

His Lys Val Tyr Ala Cys Glu Val Thr His Gln Gly Leu Ser Ser Pro
        195             200             205

Val Thr Lys Ser Phe Asn Arg Gly Glu Cys
    210             215
```

<210> 15
<211> 448
<212> PRT
<213> Artificial Sequence

<220>
<223> heavy chain of a humanised TREM-1 antibody

```
Glu Val Gln Leu Val Glu Ser Gly Gly Gly Leu Val Gln Pro Gly Gly
1               5               10              15

Ser Leu Lys Leu Ser Cys Ala Ala Ser Gly Phe Thr Phe Ser Thr Tyr
```

                    20                          25                          30

Ala Met His Trp Val Arg Gln Ala Ser Gly Lys Gly Leu Glu Trp Val
        35                  40                  45

Gly Arg Ile Arg Thr Lys Ser Ser Asn Tyr Tyr Thr Tyr Tyr Ala Ala
        50                  55                  60

Ser Val Lys Gly Arg Phe Thr Ile Ser Arg Asp Asp Ser Lys Asn Thr
65                  70                  75                  80

Ala Tyr Leu Gln Met Asn Ser Leu Lys Thr Glu Asp Thr Ala Val Tyr
                85                  90                  95

Tyr Cys Thr Arg Asp Met Gly Ile Arg Arg Gln Phe Ala Tyr Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Cys Ser Arg Ser Thr Ser Glu Ser Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
            165                 170                 175

Val Leu Gln Ser Ser Gly Leu Tyr Ser Leu Ser Ser Val Val Thr Val
        180                 185                 190

Pro Ser Ser Ser Leu Gly Thr Lys Thr Tyr Thr Cys Asn Val Asp His
    195                 200                 205

Lys Pro Ser Asn Thr Lys Val Asp Lys Arg Val Glu Ser Lys Tyr Gly
    210                 215                 220

Pro Pro Cys Pro Pro Cys Pro Ala Pro Glu Phe Leu Gly Gly Pro Ser
225                 230                 235                 240

Val Phe Leu Phe Pro Pro Lys Pro Lys Asp Thr Leu Met Ile Ser Arg
            245                 250                 255

Thr Pro Glu Val Thr Cys Val Val Val Asp Val Ser Gln Glu Asp Pro
            260                 265                 270

```
Glu Val Gln Phe Asn Trp Tyr Val Asp Gly Val Glu Val His Asn Ala
        275                 280             285

Lys Thr Lys Pro Arg Glu Glu Gln Phe Asn Ser Thr Tyr Arg Val Val
        290                 295             300

Ser Val Leu Thr Val Leu His Gln Asp Trp Leu Asn Gly Lys Glu Tyr
305             310             315                 320

Lys Cys Lys Val Ser Asn Lys Gly Leu Pro Ser Ser Ile Glu Lys Thr
            325                 330                 335

Ile Ser Lys Ala Lys Gly Gln Pro Arg Glu Pro Gln Val Tyr Thr Leu
        340                 345             350

Pro Pro Ser Gln Glu Glu Met Thr Lys Asn Gln Val Ser Leu Thr Cys
        355                 360             365

Leu Val Lys Gly Phe Tyr Pro Ser Asp Ile Ala Val Glu Trp Glu Ser
    370             375             380

Asn Gly Gln Pro Glu Asn Asn Tyr Lys Thr Thr Pro Pro Val Leu Asp
385             390             395                 400

Ser Asp Gly Ser Phe Phe Leu Tyr Ser Arg Leu Thr Val Asp Lys Ser
            405                 410                 415

Arg Trp Gln Glu Gly Asn Val Phe Ser Cys Ser Val Met His Glu Ala
        420                 425             430

Leu His Asn His Tyr Thr Gln Lys Ser Leu Ser Leu Ser Leu Gly Lys
        435                 440             445
```

<210> 16
<211> 448
<212> PRT
<213> Artificial Sequence

<220>
<223> heavy chain of a humanised TREM-1 antibody

```
Glu Val Gln Leu Val Glu Ser Gly Gly Gly Leu Val Gln Pro Gly Gly
1               5               10              15

Ser Leu Lys Leu Ser Cys Ala Ala Ser Gly Phe Thr Phe Ser Thr Tyr
            20              25              30

Ala Met His Trp Val Arg Gln Ala Ser Gly Lys Gly Leu Glu Trp Val
```

                    35                        40                        45

        Gly Arg Ile Arg Thr Lys Ser Ser Asn Tyr Tyr Thr Tyr Tyr Ala Ala
            50              55              60

        Ser Val Lys Gly Arg Phe Thr Ile Ser Arg Asp Asp Ser Lys Asn Thr
        65              70              75              80

        Ala Tyr Leu Gln Met Asn Ser Leu Lys Thr Glu Asp Thr Ala Val Tyr
                    85              90              95

        Tyr Cys Thr Arg Asp Met Gly Ile Arg Arg Gln Phe Ala Tyr Trp Gly
                    100             105             110

        Gln Gly Thr Leu Val Thr Val Ser Ser Ala Ser Thr Lys Gly Pro Ser
                    115             120             125

        Val Phe Pro Leu Ala Pro Cys Ser Arg Ser Thr Ser Glu Ser Thr Ala
            130             135             140

        Ala Leu Gly Cys Leu Val Lys Asp Tyr Phe Pro Glu Pro Val Thr Val
        145             150             155             160

        Ser Trp Asn Ser Gly Ala Leu Thr Ser Gly Val His Thr Phe Pro Ala
                    165             170             175

        Val Leu Gln Ser Ser Gly Leu Tyr Ser Leu Ser Ser Val Val Thr Val
                    180             185             190

        Pro Ser Ser Ser Leu Gly Thr Lys Thr Tyr Thr Cys Asn Val Asp His
                    195             200             205

        Lys Pro Ser Asn Thr Lys Val Asp Lys Arg Val Glu Ser Lys Tyr Gly
            210             215             220

        Pro Pro Cys Pro Pro Cys Pro Ala Pro Glu Phe Leu Gly Gly Pro Ser
        225             230             235             240

        Val Phe Leu Phe Pro Pro Lys Pro Lys Asp Thr Leu Met Ile Ser Arg
                    245             250             255

        Thr Pro Glu Val Thr Cys Val Val Val Asp Val Ser Gln Glu Asp Pro
                    260             265             270

        Glu Val Gln Phe Asn Trp Tyr Val Asp Gly Val Glu Val His Asn Ala
                    275             280             285

41

```
Lys Thr Lys Pro Arg Glu Glu Gln Phe Asn Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Gly Leu Pro Ser Ser Ile Glu Lys Thr
                325                 330                 335

Ile Ser Lys Ala Lys Gly Gln Pro Arg Glu Pro Gln Val Tyr Thr Leu
            340                 345                 350

Pro Pro Ser Gln Glu Glu Met Thr Lys Asn Gln Val Ser Leu Thr Cys
            355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Arg Leu Thr Val Asp Lys Ser
                405                 410                 415

Arg Trp Gln Glu Gly Asn Val Phe Ser Cys Ser Val Met His Glu Ala
            420                 425                 430

Leu His Asn His Tyr Thr Gln Lys Ser Leu Ser Leu Ser Leu Gly Lys
            435                 440                 445


<210>  17
<211>  233
<212>  PRT
<213>  Macaca fascicularis

<400>  17

Met Arg Lys Thr Arg Leu Trp Gly Leu Leu Trp Met Leu Phe Val Ser
1               5                   10                  15

Glu Leu Arg Ala Thr Thr Glu Leu Thr Glu Glu Lys Tyr Glu Tyr Lys
            20                  25                  30

Glu Gly Gln Thr Leu Glu Val Lys Cys Asp Tyr Ala Leu Glu Lys Tyr
            35                  40                  45

Ala Asn Ser Arg Lys Ala Trp Gln Lys Met Glu Gly Lys Met Pro Lys
        50                  55                  60
```

```
Ile Leu Ala Lys Thr Glu Arg Pro Ser Glu Asn Ser His Pro Val Gln
65              70              75                  80

Val Gly Arg Ile Thr Leu Glu Asp Tyr Pro Asp His Gly Leu Leu Gln
                85              90                  95

Val Gln Met Thr Asn Leu Gln Val Glu Asp Ser Gly Leu Tyr Gln Cys
            100             105             110

Val Ile Tyr Gln His Pro Lys Glu Ser His Val Leu Phe Asn Pro Ile
            115             120             125

Cys Leu Val Val Thr Lys Gly Ser Ser Gly Thr Pro Gly Ser Ser Glu
            130             135             140

Asn Ser Thr Gln Asn Val Tyr Arg Thr Pro Ser Thr Thr Ala Lys Ala
145             150             155                 160

Leu Gly Pro Arg Tyr Thr Ser Pro Arg Thr Val Thr Gln Ala Pro Pro
                165             170                 175

Glu Ser Thr Val Val Val Ser Thr Pro Gly Ser Glu Ile Asn Leu Thr
            180             185             190

Asn Val Thr Asp Ile Ile Arg Val Pro Val Phe Asn Ile Val Ile Ile
            195             200             205

Val Ala Gly Gly Phe Leu Ser Lys Ser Leu Val Phe Ser Val Leu Phe
    210             215             220

Ala Val Thr Leu Arg Ser Phe Gly Pro
225             230

<
```

**Description**

**TECHNICAL FIELD**

The invention is directed to TREM-1 mAbs and to specific negatively charged amino acids mutations of uncharged amino acids involved in TREM-1 mAb self-interactions in order to lower the mAb solution viscosity and retain target affinity.

**INCORPORATION-BY-REFERENCE OF THE SEQUENCE LISTING**

SEQ ID NO: 1 represents the amino acid sequence of wild type (wt) human TREM-1.

SEQ ID NO: 2 represents the amino acid sequence of the heavy chain of a humanised TREM-1 antibody (mAb 0170 of WO2013/120553).

SEQ ID NO: 3 represents the amino acid sequence of the light chain of a humanised TREM-1 antibody (mAb 0170 of WO2013/120553).

SEQ ID NO: 4 represents the amino acid sequence of the light chain of a humanised TREM-1 Fab (0317, E27Q, E97S).

SEQ ID NO: 5 represents the amino acid sequence of the light chain of a humanised TREM-1 Fab (0318, E27Q, E97Q).

SEQ ID NO: 6 represents the amino acid sequence of the light chain of a humanised TREM-1 Fab (0319, E97S).

SEQ ID NO: 7 represents the amino acid sequence of the light chain of a humanised TREM-1 Fab (0320, E97Q)

SEQ ID NO: 8 represents the amino acid sequence of the light chain of a humanised TREM-1 Fab (0321, E27Q)

SEQ ID NO: 9 represents the amino acid sequence of the light chain of a humanised TREM-1 Fab (0322, F32A)

SEQ ID NO: 10 represents the amino acid sequence of the light chain of a humanised TREM-1 Fab (0323, F32S)

SEQ ID NO: 11 represents the amino acid sequence of the heavy chain of a humanised TREM-1 Fab (0324, A59Y)

SEQ ID NO: 12 represents the amino acid sequence of the heavy chain of a humanised TREM-1 Fab (0325, N57S)

SEQ ID NO: 13 represents the amino acid sequence of the heavy chain of a humanised TREM-1 Fab (0326, A59Y, N57S)

SEQ ID NO: 14 represents the amino acid sequence of the heavy chain of a humanised TREM-1 Fab (330, F32A, E27Q, E97Q)

SEQ ID NO: 15 represents the amino acid sequence of the heavy chain of a humanised TREM-1 Fab (A59Y) (332 is a mAb combined of SEQ ID NO: 15 as the HC and SEQ ID NO 5 as the LC)

SEQ ID NO: 16 represents the amino acid sequence of the heavy chain of a humanised TREM-1 Fab (A59Y) (333 is a mAb combined of SEQ ID NO: 16 as the HC and SEQ ID NO 14 as the LC)

SEQ ID NO: 17 represents the amino acid sequence of full length cTREM-1.

**BACKGROUND**

TREM-1 is an activating receptor expressed on monocytes, macrophages and neutrophils. These cells play a central role in chronic inflammatory diseases by releasing cytokines and other mediators that drive inflammation. TREM-1 mRNA and protein expression is up-regulated in patients with RA and IBD, and TREM-1-positive cells accumulate at sites of inflammation, correlating with disease severity. Peptidoglycan-recognition-protein 1 (PGLYRP1) expressed primarily by activated neutrophils is a ligand for TREM-1 and mediate TREM-1 signalling upon binding.

*In vitro*, engagement of TREM-1 triggers secretion of pro-inflammatory cytokines including TNF, IL-8, and monocyte chemotactic protein-1. In addition, TREM-1 signalling synergizes with multiple Toll-like Receptors (TLR) to further boost pro-inflammatory signals. In turn, this up-regulates expression of TREM-1, leading to a vicious cycle amplifying the inflammation. Increasing evidence indicates that TLRs contribute to the development and progression of chronic inflammatory diseases such as RA and IBD.

WO2013/120553 discloses humanized anti-TREM-1 mAbs which inhibit both human and cynomolgus TREM-1 function. However, the viscosity profile of anti-TREM-1 mAbs may hamper the manufacturing process to produce a drug product at >50 mg/ml and could limit the optimal dose setting in the clinic.

Charge distribution of mAbs has been studied with regards to the effect on the viscosity behaviour of mAb solutions (Ydav et al., Mol. Pharmaceutics 2012, 9, 791-802). Also, weak non-specific charge interactions that persist in dilute solutions govern the viscosity of concentrated mAb solutions (Connolly et al.,Biophys. J., 2012, 103, 69-78.).

## SUMMARY

A primary aspect of the invention is directed to an anti-TREM-1 mAb with a viscosity profile in the range expected for monomeric mAbs and which blocks TREM-1 function as potently as mAb0170 of WO2013/120553. Further to the advantageous viscosity profiles of the variant mAbs of the invention, said variants do not show agonism to the TREM-1 receptor.

One aspect of the invention is directed to an antibody or fragment thereof that is capable of binding to and blocking TREM-1, characterized in that the antibody or an antibody fragment of said antibody, has a viscosity of less than 5 cP at a concentration of 80 mg/mL, preferably less than 4 cP.

A further aspect of the invention is directed to an antibody or fragment thereof that is capable of specifically binding to and blocking TREM-1 of SEQ. ID. NO: 1 and comprises variants of SEQ. ID. NO: 2 or SEQ. ID. NO: 3 or both, wherein the variants are selected from the group consisting of 'Fab-Fab interaction' mutations, 'Fab-TREM-1 interaction' mutations and 'charge-patch" mutations of SEQ. ID. NO: 2 or SEQ. ID. NO: 3.

An antibody or fragment thereof wherein the negatively charged residues in CDR1 and CDR3 regions of SEQ. ID. NO 3 are substituted with uncharged amino acid residues.

An antibody or fragment thereof according to any of the embodiments of the invention comprising 'Fab-TREM-1 interaction' mutations, such as wherein a Phe residue of SEQ. ID. NO: 2 is substituted with Ala or Ser.

An antibody or fragment thereof according to any of the preceding claims, comprising a variant of SEQ. ID. NO 3 wherein any one of 'charge patch' residues D1, D27C, D29, D70, D94, E27, E97 of SEQ. ID. NO 3 is mutated to an amino acid residue selected from the group consisting of glycine, alanine, serine, asparagine, glutamine, threonine, cysteine, and tyrosine.

An antibody or fragment thereof comprising any one of SEQ ID NOs: 4 to 16. 322, 323 have same affinity for cynomolgus as for human TREM-1. This is advantageous.

## BRIEF DESCRIPTION OF DRAWINGS

Figure 1 depicts the viscosity profile of anti-TREM-1 mAb variants

Figure 2 depicts the ability of mAb 0170 variants to inhibit human TREM-1 signalling in human TREM-1 reporter cell line (BWZ'36/hTREM-1) stimulated with PGN and (A) recombinant PGLYRP1 or (B) PGLYRP1 expressed by activated neutrophils (average of N donors).

Figure 3 depicts the ability of mAb 0170 variants to inhibit cynomolgus TREM-1 signalling in cynomolgus TREM-1 reporter cell line (TE426.27) stimulated with PGN and recombinant PGLYRP1.

Figure 4 depicts the ability of mAb 0170 variants to inhibit TNFa release from hypoxic M2 macrophages stimulated with PGN and recombinant PGLYRP1.

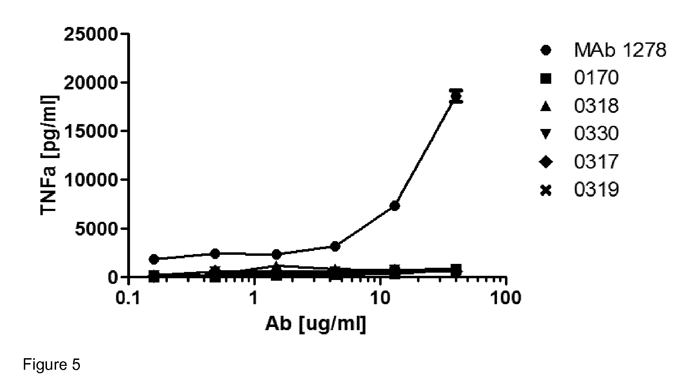

Figure 5 depicts the agonistic potential of plate-bound of mAb 0170 variants to induce TNF release from hypoxic M2 macrophages. MAb 1278 (R&D) was used as positive control.

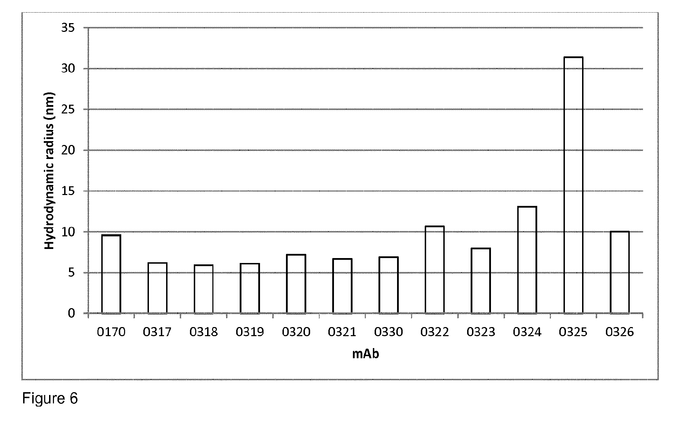

Figure 6 depicts the hydrodynamic radius of the anti-TREM-1 mAb variants compared to mAb 0170.

## DESCRIPTION

TREM-1 is a transmembrane protein that consists of 234 amino acids, including a single extracellular immunoglobulin domain and a short cytoplasmic tail with no apparent signaling motif. When activated, TREM-1 associates with the ITAM-containing signaling adaptor protein, DAP12. Downstream signalling may include activation of the NFAT transcription factor, causing an upregulation of pro-inflammatory cytokine production.

The present invention relates to antibodies that are capable of specifically binding and blocking the function of TREM-1. Antibodies of the invention may block TREM-1 function by reducing/blocking TREM-1 activation and downstream signalling.

Antibodies according to the invention may block TREM-1 by means of one of one or a combination of several different mechanisms, blocking TREM-1 directly or indirectly. For example, antibodies of the invention may prevent the natural ligand of TREM-1, peptidoglycan recognition protein 1 (PGLYRP1), from creating a functional complex with TREM-1 and/or antibodies of the invention may block TREM-1 by preventing individual TREM-1 molecules from forming dimers or multimers. TREM-1 dimerisation or multimerisation may be reduced or prevented by TREM-1 antibodies that are capable of binding to a portion of TREM-1 that would otherwise reside in the interface of a TREM-1 dimer, thus preventing individual TREM-1 molecules from associating with one another.

TREM-1 dimerisation or multimerisation may be reduced or prevented by TREM-1 antibodies that interfere with the interaction of TREM-1 with its ligand. Antibodies according to the current invention may block PGLYRP1-induced activation of TREM-1. PGLYRP1, a highly conserved, 196 amino acid long protein consisting of a signal peptide and a peptidoglycan binding domain, is expressed in neutrophils and released upon their activation. Antibodies according to the current invention may down-regulate pro-inflammatory cytokine release from myeloid cells. Antibodies according to the current invention may block the release of TNF, MIP-1beta, MCP-1, IL-1beta, GM.CSF, IL-6 and/or IL-8 from macrophages, neutrophils, synovial tissue cells and/or a reporter cell, as disclosed herein.

Antibodies of the invention may be capable of binding both human TREM-1 and TREM-1 from another species than a human being. The term "TREM-1", as used herein, thus encompasses any naturally occurring form of TREM-1 which may be derived from any suitable organism. For example, TREM-1 for use as described herein may be vertebrate TREM-1, such as mammalian TREM-1, such as TREM-1 from a primate (such as a human, a chimpanzee, a cynomolgus monkey or a rhesus monkey); a rodent (such as a mouse or a rat), a lagomorph (such as a rabbit), or an artiodactyl (such a cow, sheep, pig or camel). Preferably, the TREM-1 is SEQ ID NO: 1 (human TREM-1). The TREM-1 may be a mature form of TREM-1 such as a TREM-1 protein that has undergone post-translational processing within a suitable cell. Such a mature TREM-1 protein may, for example, be glycosylated. The TREM-1 may be a full length TREM-1 protein.

Antibodies of the invention may be monoclonal antibodies, in the sense that they are directly or indirectly derived from a single clone of a B lymphocyte. TREM-1 antibodies may be produced, screened and purified using, for example, the methods described in the Examples of WO2013/120553. In brief, a suitable mouse such as a TREM-1 or TREM-1/TREM-3 knock-out (KO) mouse may be immunised with TREM-1, a cell expressing TREM-1 or a combination of both.

Antibodies of the invention may be polyclonal in the sense of being a mixture of monoclonal antibodies according to the current invention.

Primary screening of hybridoma supernatants may be performed using direct ELISA or FMAT and secondary screening may be performed using flow cytometry. Positive hybridoma supernatants may then be screened in a reporter gene assay.

Antibodies may be recombinantly expressed in prokaryotic or eukaryotic cells. The prokaryotic cell may be *E. coli.* The eukaryotic cell may be a yeast, insect or mammalian cell, such as a cell derived from an organism that is a primate (such as a human, a chimpanzee, a cynomolgus monkey or a rhesus monkey), a rodent (such as a mouse or a rat), a lagomorph (such as a rabbit) or an artiodactyl (such a cow, sheep, pig or camel). Suitable mammalian cell lines include, but are not limited to, HEK293 cells, CHO cells and HELA cells. TREM-1 antibodies may also be produced by means of other methods known to the person skilled in the art, such as a phage display or a yeast display.

Once produced, antibodies may be screened for binding to, for example, full length TREM-1 or mutants thereof using the methods described in the Examples of WO2013/120553.

One embodiment of the current invention is a method of identifying a functional TREM-1 antibody. Antibodies that are capable of specifically binding TREM-1 and that have an effect upon TREM-1 activation and downstream signalling are herein referred to as "functional TREM-1 antibodies". A "functional" TREM-1 antibody herein refers to an antibody that is capable of blocking or stimulating TREM-1. The method of identifying a functional TREM-1 antibody comprises (a) culturing a first cell expressing TREM-1, a signalling protein and a reporter construct; (b) measuring the activity of the first cell when said cell is incubated with a TREM-1 modifying agent; (c) contacting the co-culture of (b) with a TREM-1 antibody; and (d) measuring that the activity of the first cell is less than or more than the activity measured in (b).

The "first cell" of (a) may be a cell of haematopoetic origin, such as a myeloid cell, such as a T-cell. The signalling protein of (a) may be any signalling protein that is capable of forming a complex with TREM-1. Suitable signalling proteins include DAP10, DAP12, TCR zeta, Fc gamma RIII and an Fc receptor, or part thereof. The reporter construct of (a) may be any construct that is capable of being activated via the signalling protein and generating a recognisable signal. Suitable reporter constructs comprise a transcription factor and a reporter gene. The signalling protein may signal via a transcription factor selected from the group consisting of the NFAT and NFκB. The reporter gene is a gene that is not natively expressed in said first cell and may be a gene that encodes β-galactosidase, luciferase, green flourescent protein (GFP) or chloramphenicol transferase. Said first cell may be transfected with a transcription factor and a reporter gene using methods that are well known in the art.

The "BWZ/hTREM-1 reporter cell" and "TE426.27 reporter cell" described in the Examples is one example of a "first cell".

The modifying agent of (b) may be a TREM-1 ligand or an activated neutrophil. The "TREM-1 antibody" of (c) may be a TREM-1 specific hybridoma supernatant or a purified antibody. The activity measured in (d) is the signal produced by the reporter construct. An example of such signalling is the luminescence caused by NFAT-driven LacZ (β-lactamase luciferase) production.

The method may be tailored to identify a blocking TREM-1 antibody. The method of identifying a blocking TREM-1 antibody comprises (a) culturing a first cell expressing TREM-1, a signalling protein and a reporter construct; (b) measuring the activity of the first cell when said cell is incubated with an activated neutrophil; (c) contacting the co-culture of the first cell and the activated neutrophil with a TREM-1 antibody; and (d) measuring that the activity of the first cell is less than the activity measured in (b).

The method may also be tailored to identify a stimulating TREM-1 antibody. The method of identifying a stimulating TREM-1 antibody comprises (a) culturing a first cell expressing TREM-1, a signalling protein and a reporter construct; (b) measuring the activity of the first cell; (c) contacting/incubating said cell with a TREM-1 antibody; and (d) measuring that the activity of the first cell is more than the activity of the measured in (b).

The present invention relates to blocking TREM-1 antibodies that may be identified by means of the method, herein disclosed, of identifying a blocking antibody. When tested using the method described above and in the Examples, an antibody according to the current invention may, at a concentration of less than 50 μg/ml, such as less than 40 μg/ml, such as less than 30 μg/ml, such as less than 20 μg/ml, such as less than 10 μg/ml, such as less than 5 μg/ml, such as less than 1 μg/ml - be capable of reducing the activity of said first cell by 50%, such as 60%, such as 70%, such as 80%, such as 90%, such as 95%, such as 100%. An antibody according to the invention may be capable of completely extinguishing the activity of the first cell. When tested using the method described above and in the Examples, an antibody according to the current invention may, at a concentration of less than 1 μg/ml - such as less than 0.9 μg/ml, such as less than 0.8 μg/ml, such as less than 0.7 μg/ml, such as less than 0.6 μg/ml, such as less than 0.5 μg/ml, such as less than 0.4 μg/ml, such as less than 0.3 μg/ml, such as less than 0.2 μg/ml - be capable of extinguishing the activity of the first cell.

The present invention also relates to blocking TREM-1 antibodies that may be identified by other means than the method herein disclosed.

The term "antibody" herein refers to a protein, derived from a germline immunoglobulin sequence, which is capable of specifically binding to an antigen (TREM-1) or a portion thereof. The term includes full length antibodies of any class or isotype (that is, IgA, IgE, IgG, IgM and/or IgY) and any single chain or fragment thereof. An antibody that specifically binds to an antigen, or portion thereof, may bind exclusively to that antigen, or portion thereof, or it may bind to a limited number of homologous antigens, or portions thereof. Full-length antibodies usually comprise at least four polypeptide chains: two heavy (H) chains and two light (L) chains that are interconnected by disulfide bonds. One immunoglobulin sub-class of particular pharmaceutical interest is the IgG family. In humans, the IgG class may be subdivided into 4 sub-classes: IgG1, IgG2, IgG3 and IgG4, based on the sequence of their heavy chain constant regions. The light chains can be divided into two types, kappa and lambda, based on differences in their sequence composition. IgG molecules are composed of two heavy chains, interlinked by two or more disulfide bonds, and two light chains, each attached to a heavy chain by a disulfide bond. A heavy chain may comprise a heavy chain variable region (VH) and up to three heavy chain constant (CH) regions: CH1, CH2 and CH3. A light chain may comprise a light chain variable region (VL) and a light chain constant region (CL). VH and VL regions can be further subdivided into regions of hypervariability, termed complementarity determining regions (CDRs), interspersed with regions that are more conserved, termed framework regions (FR). VH and VL regions are typically composed of three CDRs and four FRs, arranged from amino-terminus to carboxy-terminus in the following order: FR1, CDR1, FR2, CDR2, FR3, CDR3, FR4. The hypervariable regions of the heavy and light chains form a [binding] domain that is capable of interacting with an antigen, whilst the constant region of an antibody may mediate binding of the immunoglobulin to host tissues or factors, including but not limited to various cells of the immune system (effector cells), Fc receptors and the first component (Clq) of the classical complement system.

Antibodies of the current invention may be isolated. The term "isolated antibody" refers to an antibody that has been separated and/or recovered from (an)other component(s) in the environment in which it was produced and/or that has been purified from a mixture of components present in the environment in which it was produced.

Certain antigen-binding fragments of antibodies may be suitable in the context of the current invention, as it has been shown that the antigen-binding function of an antibody can be performed by fragments of a full-length antibody. The term "antigen-binding fragment" of an antibody refers to one or more fragment(s) of an antibody that retain the ability to specifically bind to an antigen, such as TREM-1, as described herein. Examples of antigen-binding fragments include Fab, Fab', F(ab)2, F(ab')2, F(ab)S, Fv (typically the VL and VH domains of a single arm of an antibody), single-chain Fv (scFv; see e.g.. Bird et al., Science 1988; 242:42S-426; and Huston et al. PNAS 1988; 85:5879-5883), dsFv, Fd (typically the VH and CHI domain), and dAb (typically a VH domain) fragments; VH, VL, VhH, and V-NAR domains; monovalent molecules comprising a single VH and a single VL chain; minibodies, diabodies, triabodies, tetrabodies, and kappa bodies (see, e.g., Ill et al., Protein Eng 1997;10:949-57); camel IgG; IgNAR; as well as one or more isolated CDRs or a functional paratope, where the isolated CDRs or antigen-binding residues or polypeptides can be associated or linked together so as to form a functional antibody fragment. Various types of antibody fragments have been described or reviewed in, e.g., Holliger and Hudson, Nat Biotechnol 2005;2S:1126-1136; WO2005040219, and published U.S. Patent Applications 20050238646 and 20020161201. These antibody fragments may be obtained using conventional techniques known to those of skill in the art, and the fragments may be screened for utility in the same manner as intact antibodies.

An antibody of the invention may be a human antibody or a humanised antibody. The term "human antibody", as used herein, is intended to include antibodies having variable regions in which at least a portion of a framework region and/or at least a portion of a CDR region are derived from human germline immunoglobulin sequences. (For example, a human antibody may have variable regions in which both the framework and CDR regions are derived from human germline immunoglobulin sequences.) Furthermore, if the antibody contains a constant region, the constant region is also derived from human germline immunoglobulin sequences. The human antibodies of the invention may include amino acid residues not encoded by human germline immunoglobulin sequences (e.g., mutations introduced by random or site-specific mutagenesis *in vitro* or by somatic mutation *in vivo*).

Such a human antibody may be a human monoclonal antibody. Such a human monoclonal antibody may be produced by a hybridoma which includes a B cell obtained from a transgenic nonhuman animal, e.g., a transgenic mouse, having a genome comprising a human heavy chain transgene and a light chain transgene fused to an immortalized cell.

Human antibodies may be isolated from sequence libraries built on selections of human germline sequences, further diversified with natural and synthetic sequence diversity.

Human antibodies may be prepared by *in vitro* immunisation of human lymphocytes followed by transformation of the lymphocytes with Epstein-Barr virus.

The term "human antibody derivative" refers to any modified form of the human antibody, such as a conjugate of the antibody and another agent or antibody.

The term "humanised antibody", as used herein, refers to a human/non-human chimeric antibody that contains one or more sequences (CDR regions or parts thereof) that are derived from a non-human immunoglobulin. A humanised antibody is, thus, a human immunoglobulin (recipient antibody) in which at least residues from a hyper-variable region of the recipient are replaced by residues from a hyper-variable region of an antibody from a non-human species (donor antibody) such as from a mouse, rat, rabbit or non-human primate, which have the desired specificity, affinity, sequence composition and functionality. In some instances, FR residues of the human immunoglobulin are replaced by corresponding non-human residues. An example of such a modification is the introduction of one or more so-called back-mutations, which are typically amino acid residues derived from the donor antibody. Humanisation of an antibody may be carried out using recombinant techniques known to the person skilled in the art (see, e.g., Antibody Engineering, Methods in Molecular Biology, vol. 248, edited by Benny K. C. Lo). A suitable human recipient framework for both the light and heavy chain variable domain may be identified by, for example, sequence or structural homology. Alternatively, fixed recipient frameworks may be used, e.g., based on knowledge of structure, biophysical and biochemical properties. The recipient frameworks can be germline derived or derived from a mature antibody sequence. CDR regions from the donor antibody can be transferred by CDR grafting. The CDR grafted humanised antibody can be further optimised for e.g. affinity, functionality and biophysical properties by identification of critical framework positions where re-introdution (backmutation) of the amino acid residue from the donor antibody has beneficial impact on the properties.of the humanised antibody. In addition to donor antibody derived backmutations, the humanised antibody can be engineered by introduction of germline residues in the CDR or framework regions, elimination of immunogenic epitopes, site-directed mutagenesis, affinity maturation, etc.

Furthermore, humanised antibodies may comprise residues that are not found in the recipient antibody or in the donor antibody. These modifications are made to further refine antibody performance. In general, a humanised antibody will comprise at least one - typically two - variable domains, in which all or substantially all of the CDR regions correspond to those of a non-human immunoglobulin and in which all or substantially all of the FR residues are those of a human immunoglobulin sequence. The humanised antibody can, optionally, also comprise at least a portion of an immunoglobulin constant region (Fc), typically that of a human immunoglobulin.

The term "humanised antibody derivative" refers to any modified form of the humanised antibody, such as a conjugate of the antibody and another agent or antibody.

The term "chimeric antibody", as used herein, refers to an antibody whose light and heavy chain genes have been constructed, typically by genetic engineering, from immunoglobulin variable and constant region genes that originate from different species. For example, the variable segments of genes from a mouse monoclonal antibody may be joined to human constant segments.

The fragment crystallisable region ("Fc region"/"Fc domain") of an antibody is the N-terminal region of an antibody, which comprises the constant CH2 and CH3 domains. The Fc domain may interact with cell surface receptors called Fc receptors, as well as some proteins of the complement system. The Fc region enables antibodies to interact with the immune system. In one aspect of the invention, antibodies may be engineered to include modifications within the Fc region, typically to alter one or more of its functional properties, such as serum half-life, complement fixation, Fc-receptor binding, protein stability and/or antigen-dependent cellular cytotoxicity, or lack thereof, among others. Furthermore, an antibody of the invention may be chemically modified (e.g., one or more chemical moieties can be attached to the antibody) or be modified to alter its glycosylation, again to alter one or more functional properties of the antibody. An IgG1 antibody may carry a modified Fc domain comprises one or more, and perhaps all of the following mutations that will result in decreased affinity to certain Fc receptors (L234A, L235E, and G237A) and in reduced C1q-mediated complement fixation (A330S and P331S), respectively (residue numbering according to the EU index).

The isotype of an antibody of the invention may be IgG, such as IgG1, such as IgG2, such as IgG4. If desired, the class of an antibody may be "switched" by known techniques. For example, an antibody that was originally produced as an IgM molecule may be class switched to an IgG antibody. Class switching techniques also may be used to convert one IgG subclass to another, for example: from IgG1 to IgG2 or IgG4; from IgG2 to IgG1 or IgG4; or from IgG4 to IgG1 or IgG2. Engineering of antibodies to generate constant region chimeric molecules, by combination of regions from different IgG subclasses, can also be performed.

In one embodiment, the hinge region of CH1 is modified such that the number of cysteine residues in the hinge region is altered, e.g., increased or decreased. This approach is described further for instance in U.S. Patent No. 5,677,425 by Bodmer et al.

The constant region may be modified to stabilize the antibody, e.g., to reduce the risk of a bivalent antibody separating into two monovalent VH-VL fragments. For example, in an IgG4 constant region, residue S228 (residue numbering according to the EU index) may be mutated to a proline (P) residue to stabilise inter heavy chain disulphide bridge formation at the hinge (see, e.g., Angal et al., Mol Immunol. 1995; 30: 105-8).

Antibodies or fragments thereof may also be defined in terms of their complementarity-determining regions (CDRs). The term "complementarity-determining region" or "hypervariable region", when used herein, refers to the regions of an antibody in which amino acid residues involved in antigen binding are situated. The region of hypervariability or CDRs can be identified as the regions with the highest variability in amino acid alignments of antibody variable domains. Databases can be used for CDR identification such as the Kabat database, the CDRs e.g. being defined as comprising amino acid residues 24-34 (L1), 50-59 (L2) and 89-97 (L3) of the light-chain variable domain and 31-35 (H1), 50-65 (H2) and 95-102 (H3) in the heavy-chain variable domain; (Kabat et al. (1991) Sequences of Proteins of Immunological Interest, Fifth Edition, U.S. Department of Health and Human Services, NIH Publication No. 91-3242) Alternatively CDRs can be defined as those residues from a "hypervariable loop" (residues 26-33 (L1), 50-52 (L2) and 91-96 (L3) in the light-chain variable domain and 26-32 (H1), 53-55 (H2) and 96-101 (H3) in the heavy-chain variable domain; Chothia and Lesk, J. Mol. Biol 1987; 196: 901-917). Typically, the numbering of amino acid residues in this region is performed by the method described in Kabat et al., supra. Phrases such as "Kabat position", "Kabat residue", and "according to Kabat" herein refer to this numbering system for heavy chain variable domains or light chain variable domains. Using the Kabat numbering system, the actual linear amino acid sequence of a peptide may contain fewer or additional amino acids corresponding to a shortening of, or insertion into, a framework (FR) or CDR of the variable domain. For example, a heavy chain variable domain may include amino acid insertions (residue 52a, 52b and 52c according to Kabat) after residue 52 of CDR H2 and inserted residues (e.g. residues 82a, 82b, and 82c, etc. according to Kabat) after heavy chain FR residue 82. The Kabat numbering of residues may be determined for a given antibody by alignment at regions of homology of the sequence of the antibody with a "standard" Kabat numbered sequence.

The term "framework region" or "FR" residues refer to those VH or VL amino acid residues that are not within the CDRs, as defined herein.

The mAb 0170 antibody has a variable heavy chain sequence as shown in SEQ ID NO: 2 and a variable light chain sequence as shown in SEQ ID NO: 3. An antibody of the invention may comprise this variable heavy chain sequence and/or this variable light chain sequence. The mAb 0170 antibody has the CDR sequences shown at amino acids 31 to 35, 50 to 68 and 101 to 110 of SEQ ID NO: 2 and amino acids 24 to 38, 54 to 60 and 93 to 101 of SEQ ID NO: 3.

The heavy chain of an antibody according to the invention may comprise a CDR1 sequence of amino acids 31 to 35 (TYAMH) of SEQ ID NO: 2, wherein one of these amino acids may be substituted by a different amino acid.

The heavy chain of an antibody according to the invention may comprise a CDR2 sequence of amino acids 50 to 68 (RIRTKSSNYATYYADSVKD) of SEQ ID NO: 2, wherein one, two or three of these amino acids may be substituted by a different amino acid.

The heavy chain of an antibody according to the invention may comprise a CDR3 sequence of amino acids 101 to 110 (DMGQRRQFAY) of SEQ ID NO: 2, wherein one, two or three of these amino acids may be substituted by a different amino acid.

The light chain of an antibody according to the invention may comprise a CDR1 sequence of amino acids 24 to 38 (RASESVDTFDYSFLH) of SEQ ID NO: 3, wherein one, two or three of these amino acids may be substituted with a different amino acid.

The light chain of an antibody according to the invention may comprise a CDR2 sequence of amino acids 54 to 60 (RASNLES) of SEQ ID NO: 3, wherein one or two of these amino acids may be substituted with a different amino acid.

The light chain of an antibody according to the invention may comprise a CDR3 sequence of amino acids 93 to 101 (QQSNEDPYT) of SEQ ID NO: 3, wherein one or two of these amino acids may be substituted with a different amino acid.

The mAb 0170 antibody has a heavy chain sequence as shown in SEQ ID NO: 2 and a light chain sequence as shown in SEQ ID NO: 3. An antibody of the invention may comprise this heavy chain sequence or this light chain sequence. Either the heavy or light chain of the antibody of the invention, or both, may be a variant of mAb 0170. The mAb 0170 antibody has the CDR sequences shown at amino acids 31 to 35, 50 to 68 and 101 to 110 of SEQ ID NO: 2 and amino acids 24 to 38, 54 to 60 and 93 to 101 of SEQ ID NO: 3. An antibody of the invention may comprise 1, 2, 3, 4, 5 or all 6 of these CDR sequences.

The heavy chain of an antibody according to the invention may comprise a CDRH3 sequence of amino acids 101 to 110 (DMGIRRQFAY) of SEQ ID NO: 2, wherein one, two or three of these amino acids may be substituted by a different amino acid.

The term "antigen" (Ag) refers to the molecular entity used for immunization of an immunocompetent vertebrate to produce the antibody (Ab) that recognizes the Ag. Herein, Ag is termed more broadly and is generally intended to include target molecules that are specifically recognized by the Ab, thus including fragments or mimics of the molecule used in the immunization process, or other process, e.g. phage display, used for generating the Ab.

The term "epitope", as used herein, is defined in the context of a molecular interaction between an "antigen binding polypeptide", such as an antibody (Ab), and its corresponding antigen (Ag). Generally, "epitope" refers to the area or region on an Ag to which an Ab specifically binds, i.e. the area or region in physical contact with the Ab. Physical contact may be defined through various criteria (e.g. a distance cut-off of 2-6Å, such as 3Å, such as 4 Å, such as 5Å; or solvent accessibility) for atoms in the Ab and Ag molecules. A protein epitope may comprise amino acid residues in the Ag that are directly involved in binding to a Ab (also called the immunodominant component of the epitope) and other amino acid residues, which are not directly involved in binding, such as amino acid residues of the Ag which are effectively blocked by the Ab, i.e. amino acid residues within the "solvent-excluded surface" and/or the "footprint" of the Ab.

The term epitope herein comprises both types of binding region in any particular region of TREM-1 that specifically binds to a TREM-1 antibody. TREM-1 may comprise a number of different epitopes, which may include, without limitation, conformational epitopes which consist of one or more non-contiguous amino acids located near each other in the mature TREM-1 conformation and post-translational epitopes which consist, either in whole or part, of molecular structures covalently attached to TREM-1, such as carbohydrate groups.

The epitope for a given antibody (Ab)/antigen (Ag) pair can be described and characterized at different levels of detail using a variety of experimental and computational epitope mapping methods. The experimental methods include mutagenesis, X-ray crystallography, Nuclear Magnetic Resonance (NMR) spectroscopy, Hydrogen deuterium eXchange Mass Spectrometry (HX-MS) and various competition binding methods; methods that are known in the art. As each method relies on a unique principle, the description of an epitope is intimately linked to the method by which it has been determined. Thus, depending on the epitope mapping method employed, the epitope for a given Ab/Ag pair may be described differently.

At its most detailed level, the epitope for the interaction between the Ag and the Ab can be described by the spatial coordinates defining the atomic contacts present in the Ag-Ab interaction, as well as information about their relative contributions to the binding thermodynamics. At a less detailed level, the epitope can be characterized by the spatial coordinates defining the atomic contacts between the Ag and Ab. At an even less detailed level the epitope can be characterized by the amino acid residues that it comprises as defined by a specific criteria such as the distance between or solvent accessibility of atoms in the Ab:Ag complex. At a further less detailed level the epitope can be characterized through function, e.g. by competition binding with other Abs. The epitope can also be defined more generically as comprising amino acid residues for which substitution by another amino acid will alter the characteristics of the interaction between the Ab and Ag.

In the context of an X-ray derived crystal structure defined by spatial coordinates of a complex between an Ab, e.g. a Fab fragment, and its Ag, the term epitope is herein, unless otherwise specified or contradicted by context, specifically defined as TREM-1 residues characterized by having a heavy atom (i.e. a non-hydrogen atom) within a distance of, eg., 4 Å from a heavy atom in the Ab.

From the fact that descriptions and definitions of epitopes, dependant on the epitope mapping method used, are obtained at different levels of detail, it follows that comparison of epitopes for different Abs on the same Ag can similarly be conducted at different levels of detail.

Epitopes described on the amino acid level, e.g. determined from an X-ray structure, are said to be identical if they contain the same set of amino acid residues. Epitopes are said to overlap if at least one amino acid is shared by the epitopes. Epitopes are said to be separate (unique) if no amino acid residue are shared by the epitopes.

Epitopes may also be defined indirectly, by means of comparing the binding kinetics of antibodies to wild type human TREM-1 with those of human TREM-1 variants that have alanine mutations in anticipated epitopes. Decreased affinity or abrogated binding of an antibody to variants of human TREM-1 in which an amino acid residue has been replaced with an alanine residue indicates that the mutated amino acid contributes to the interaction between said antibody and wild type human TREM-1. This approach provides a negative identification of the epitope. The method is compromised in effectively defining the epitope by the fact that protein misfolding or unfolding would give similar results as abrogation of interaction. The analysis can be complemented by comparative gain of function mutational analyses of an orthologous target protein (eg., cynomolgus monkey TREM-1), if a cross-reactive antibody exists. The comparison will define the epitope differences between the antibody that does not cross-react with, eg., cynomolgus monkey TREM-1 and the cross-reactive antibody.

Indirect identification of the epitope can also be provided by means of measuring antibody (or antibody fragment) binding to variants of the wild type antigen (TREM-1). If an antibody or fragment thereof binds, eg., human but not cynomolgus monkey TREM-1 and if said antibody or fragment thereof is capable of binding a partly humanised variant of cynomolgus monkey TREM-1 then this regained binding indicates that the substituted amino acid residue(s) is/are important for the interaction of the antibody with the antigen. In the same way, increased affinity for humanized variants of cynomolgus monkey TREM-1, of an anti-human TREM-1 antibody (or its Fab fragment) that has a weaker binding to cynomolgus monkey TREM-1 compared to human TREM-1, can provide information on the identity of residues composing the binding epitope.

The effect of the same mutations on any given cross-reactive antibody makes it possible to discriminate between possible protein misfolding (abrogated binding to both antibodies) and loss of interaction in human TREM-1 (binding to one of the antibodies and abrogated binding to the other antibody), whilst unambiguously providing information on the epitope differences between the antibody that does not cross-react and the cross reactive antibody on an amino acid level.

Antibodies of the current invention may be capable of binding variants of human TREM-1 as determined using, eg., surface plasmon resonance.

Antibodies of the current invention may be capable of binding variants of cynomolgus monkey TREM-1 as determined using, eg., surface plasmon resonance.

An antibody of the invention may be capable of specifically binding TREM-1, wherein said antibody is capable of specifically binding (i) at least one amino acid residue selected from the group consisting of the A21, T22, K23, L24, T25, E26, and (ii) at least one amino acid residue selected from the group consisting of the A49, S50, S51, Q52, K53, A54, W55, Q56, I57, I58, R59, D60, G61, E62, M63, P64, K65, T66, L67, A68, C69, T70, E71, R72, P73, S74, K75, N76, S77, H78, P79, V80, Q81, V82, G83, R84, I85 and (iii) at least one amino acid residue selected from the group consisting of the C113, V114, I115, Y116, Q117, P118 and P119 of human TREM-1.

An antibody of the invention may be capable of specifically binding a polypeptide comprising amino acids D38 to F48 of SEQ ID NO: 1 (human TREM-1), as determined using, eg., HX-MS or X-ray diffraction.

An antibody of the invention may have an epitope comprising one, two, three, four, five, six, seven or all of the amino acid residues D38, V39, K40, C41, D42, Y43, T44 and L45 of SEQ ID NO: 1 (human TREM-1) and one, two or all of the amino acid residues selected from the group consisting of the E46, K47 and F48 of SEQ ID NO: 1 (human TREM-1), as determined using, eg., HX-MS or X-ray diffraction.

An antibody of the invention may have an epitope comprising one, two, three or all of the amino acid residues selected from the group consisting of the D42, E46, D92 and H93 of SEQ ID NO: 1 (human TREM-1), as determined using variants of TREM-1 and surface plasmon resonance.

An antibody of the invention may have an epitope comprising at least the amino acid residues E46 and/or D92 of SEQ ID NO: 1 (human TREM-1), as determined using variants of TREM-1 and surface plasmon resonance.

An antibody of the invention may further comprise one, two or all of the amino acid residues selected from the group consisting of L31, I86 and V101 of SEQ ID NO: 1 (human TREM-1).

An antibody of the invention may be capable of specifically binding a polypeptide comprising amino acid residues E19 to L26 of cynomolgus monkey TREM-1 (SEQ ID NO: 17), as determined using, eg. HX-MS or X-ray diffraction.

An antibody of the invention may be capable of specifically binding human TREM-1, wherein the epitope of said antibody comprises one, two, three, four, five, six, seven, eight, nine or all of the amino acid residues selected from the group consisting of the the V39, K40, C41, D42, Y43, L45, E46, K47, F48 and A49 of SEQ ID NO: 1.

An antibody of the invention may be capable of specifically binding human TREM-1, wherein the epitope of said antibody comprises the D42 of SEQ ID NO: 1. An antibody of the invention may be capable of specifically binding human TREM-1, wherein the epitope of said antibody comprises the E46 of SEQ ID NO: 1. The epitope of said antibody may comprise the V39, C41, D42, Y43, L45 of SEQ ID NO: 1. The epitope of said antibody may comprise the E46, K47 and A49 of SEQ ID NO: 1. The epitope of said antibody may further comprise the F48 of SEQ ID NO: 1.

The definition of the term "paratope" is derived from the above definition of "epitope" by reversing the perspective. Thus, the term "paratope" refers to the area or region on the antibody to which an antigen specifically binds, i.e. with which it makes physical contact to the antigen.

In the context of an X-ray derived crystal structure, defined by spatial coordinates of a complex between an antibody, such as a Fab fragment, and its antigen, the term paratope is herein, unless otherwise specified or contradicted by context, specifically defined as antigen residues characterized by having a heavy atom (i.e. a non-hydrogen atom) within a distance of 4 Å, from a heavy atom in TREM-1.

The epitope and paratope for a given antibody (Ab)/antigen (Ag) pair may be identified by routine methods. For example, the general location of an epitope may be determined by assessing the ability of an antibody to bind to different fragments or variant TREM-1 polypeptides. The specific amino acids within TREM-1 that make contact with an antibody (epitope) and the specific amino acids in an antibody that make contact with TREM-1 (paratope) may also be determined using routine methods. For example, the antibody and target molecule may be combined and the Ab:Ag complex may be crystallised. The crystal structure of the complex may be determined and used to identify specific sites of interaction between the antibody and its target.

Antibodies that bind to the same antigen can be characterised with respect to their ability to bind to their common antigen simultaneously and may be subjected to "competition binding"/"binning". In the present context, the term "binning" refers to a method of grouping antibodies that bind to the same antigen. "Binning" of antibodies may be based on competition binding of two antibodies to their common antigen in assays based on standard techniques such as surface plasmon resonance (SPR), ELISA or flow cytometry.

An antibody's "bin" is defined using a reference antibody. If a second antibody is unable to bind to an antigen at the same time as the reference antibody, the second antibody is said to belong to the same "bin" as the reference antibody. In this case, the reference and the second antibody competitively bind the same part of an antigen and are coined "competing antibodies". If a second antibody is capable of binding to an antigen at the same time as the reference antibody, the second antibody is said to belong to a separate "bin". In this case, the reference and the second antibody do not competitively bind the same part of an antigen and are coined "non-competing antibodies".

Antibody "binning" does not provide direct information about the epitope. Competing antibodies, i.e. antibodies belonging to the same "bin" may have identical epitopes, overlapping epitopes or even separate epitopes. The latter is the case if the reference antibody bound to its epitope on the antigen takes up the space required for the second antibody to contact its epitope on the antigen ("steric hindrance"). Non-competing antibodies generally have separate epitopes.

An antibody of the invention may compete with mAb 0170 for binding to human TREM-1. An antibody of the invention may compete with mAb 0170 for binding to cynomolgus monkey TREM-1. In other words, an antibody of the invention may belong to the same "bin" as mAb 0170.

The term "binding affinity" herein refers to a measurement of the strength of a noncovalent interaction between two molecules, e.g. an antibody, or fragment thereof, and an antigen. The term "binding affinity" is used to describe monovalent interactions (intrinsic activity).

Binding affinity between two molecules, e.g. an antibody, or fragment thereof, and an antigen, through a monovalent interaction may be quantified by determination of the equilibrium dissociation constant ($K_D$). In turn, $K_D$ can be determined by measurement of the kinetics of complex formation and dissociation, e.g. by the SPR method. The rate constants corresponding to the association and the dissociation of a monovalent complex are referred to as the association rate constant $k_a$ (or $k_{on}$) and dissociation rate constant $k_d$ (or $k_{off}$), respectively. $K_D$ is related to $k_a$ and $k_d$ through the equation $K_D = k_d / k_a$.

Following the above definition, binding affinities associated with different molecular interactions, such as comparison of the binding affinity of different antibodies for a given antigen, may be compared by comparison of the $K_D$ values for the individual antibody/antigen complexes.

An antibody of the invention may bind human TREM-1 with an affinity (KD) that is $1 \times 10^{-7}$ M or less, $1 \times 10^{-8}$M or less, or $1 \times 10^{-9}$M or less, or $1 \times 10^{-10}$M or less, $1 \times 10^{-11}$M or less, $1 \times 10^{-12}$M or less or $1 \times 10^{-13}$M or less, as determined using surface plasmon resonance. An antibody of the invention may bind cynomolgus monkey TREM-1 with an affinity (KD) that is $1 \times 10^{-7}$M or less, $1 \times 10^{-8}$M or less, or $1 \times 10^{-9}$M or less, or $1 \times 10^{-10}$M or less, $1 \times 10^{-11}$M or less, $1 \times 10^{-12}$M or less or $1 \times 10^{-13}$M or less, as determined using surface plasmon resonance.

The term "binding specificity" herein refers to the interaction of a molecule such as an antibody, or fragment thereof, with a single exclusive antigen, or with a limited number of highly homologous antigens (or epitopes). In contrast, antibodies that are capable of specifically binding to TREM-1 are not capable of binding dissimilar molecules. Antibodies according to the invention may not be capable of binding Nkp44.

The specificity of an interaction and the value of an equilibrium binding constant can be determined directly by well-known methods. Standard assays to evaluate the ability of ligands (such as antibodies) to bind their targets are known in the art and include, for example, ELISAs, Western blots, RIAs, and flow cytometry analysis. The binding kinetics and binding affinity of the antibody also can be assessed by standard assays known in the art, such as SPR.

A competitive binding assay can be conducted in which the binding of the antibody to the target is compared to the binding of the target by another ligand of that target, such as another antibody.

In another aspect, the present invention provides compositions and formulations comprising molecules of the invention, such as the TREM-1 antibodies, polynucleotides, vectors and cells described herein. For example, the invention provides a pharmaceutical composition that comprises one or more TREM-1 antibodies of the invention, formulated together with a pharmaceutically acceptable carrier.

Accordingly, one object of the invention is to provide a pharmaceutical formulation comprising such a TREM-1 antibody which is present in a concentration from 0.25 mg/ml to 250 mg/ml, such as a concentration of from 10 to 200 mg/ml, and wherein said formulation has a pH from 2.0 to 10.0, such as a pH of from 4.0 to 8.0. The formulation may further comprise one or more of a buffer system, a preservative, a tonicity agent, a chelating agent, a stabilizer and/or a surfactant, as well as various combinations thereof. The use of preservatives, isotonic agents, chelating agents, stabilizers and surfactants in pharmaceutical compositions is well-known to the skilled person. Reference may be made to Remington: The Science and Practice of Pharmacy, 19th edition, 1995.

In one embodiment, the pharmaceutical formulation is an aqueous formulation. Such a formulation is typically a solution or a suspension, but may also include colloids, dispersions, emulsions, and multi-phase materials. The term "aqueous formulation" is defined as a formulation comprising at least 50% w/w water. Likewise, the term "aqueous solution" is defined as a solution comprising at least 50 % w/w water, and the term "aqueous suspension" is defined as a suspension comprising at least 50 % w/w water.

In another embodiment, the pharmaceutical formulation is a freeze-dried formulation, to which the physician or the patient adds solvents and/or diluents prior to use.

In a further aspect, the pharmaceutical formulation comprises an aqueous solution of such an antibody, and a buffer, wherein the antibody is present in a concentration from 1 mg/ml or above, and wherein said formulation has a pH from about 2.0 to about 10.0.

The TREM-1 antibodies of the present invention and pharmaceutical compositions comprising such antibodies may be used for the treatment of inflammatory diseases such as the following: inflammatory bowel disease (IBD), Crohns disease (CD), ulcerative colitis (UC), irritable bowel syndrome, rheumatoid arthritis (RA), psoriasis, psoriatic arthritis, systemic lupus erythematosus (SLE), lupus nephritis, type I diabetes, Grave's disease, multiple sclerosis (MS), autoimmune myocarditis, Kawasaki disease, coronary artery disease, chronic obstructive pulmonary disease, interstitial lung disease, autoimmune thyroiditis, scleroderma, systemic sclerosis, osteoarthritis, atoptic dermatitis, vitiligo, graft versus host disease, Sjogrens's syndrome, autoimmune nephritis, Goodpasture's syndrome, chronic inflammatory demyelinating polyneuropathy, allergy, asthma and other autoimmune diseases that are a result of either acute or chronic inflammation.

TREM-1 antibodies of the invention may be suitable for use in the treatment of individuals with inflammatory bowel disease. Inflammatory Bowel Disease (IBD) is a disease that may affect any part of the gastrointestinal tract from mouth to anus, causing a wide variety of symptoms. IBD primarily causes abdominal pain, diarrhea (which may be bloody), vomiting or weight loss, but may also cause complications outside of the gastrointestinal tract such as skin rashes, arthritis, inflammation of the eye, fatigue and lack of concentration. Patients with IBD can be divided into two major classes, those with ulcerative colitis (UC) and those with Crohn's disease (CD). CD generally involves the ileum and colon, it can affect any region of the intestine but is often discontinuous (focused areas of disease spread throughout the intestine). UC always involves the rectum (colonic) and is more continuous. In CD, the inflammation is transmural, resulting in abscesses, fistulas and strictures, whereas in UC, the inflammation is typically confined to the mucosa. There is no known pharmaceutical or surgical cure for Crohn's disease, whereas some patients with UC can be cured by surgical removal of the colon. Treatment options are restricted to controlling symptoms, maintaining remission and preventing relapse. Efficacy in inflammatory bowel disease in the clinic may be measured as a reduction in the Crohn's Disease Activity Index (CDAI) score for CD which is scoring scale based on laboratory tests and a quality of life questionnaire. In animal models, efficacy is mostly measured by increase in weight and also a disease activity index (DAI), which is a combination of stool consistency, weight and blood in stool.

TREM-1 antibodies of the invention may be suitable for use in the treatment of individuals with rheumatoid arthritis. Rheumatoid arthritis (RA) is a systemic disease that affects nearly if not all of the body and is one of the most common forms of arthritis. It is characterized by inflammation of the joint, which causes pain, stiffness, warmth, redness and swelling. This inflammation is a consequence of inflammatory cells invading the joints, and these inflammatory cells release enzymes that may digest bone and cartilage. As a result, this inflammation can lead to severe bone and cartilage damage and to joint deterioration and severe pain, among other physiologic effects. The involved joint can lose its shape and alignment, resulting in pain and loss of movement.

There are several animal models for rheumatoid arthritis known in the art. For example, in the collagen-induced arthritis (CIA) model, mice develop an inflammatory arthritis that resembles human rheumatoid arthritis. Since CIA shares similar immunological and pathological features with RA, this makes it a suitable model for screening potential human anti-inflammatory compounds. Efficacy in this model is measured by decrease in joint swelling. Efficacy in RA in the clinic is measured by the ability to reduce symptoms in patients which is measured as a combination of joint swelling, erythrocyte sedimentation rate, C-reactive protein levels and levels of serum factors, such as anti-citrullinated protein antibodies.

TREM-1 antibodies of the invention may be suitable for use in the treatment of individuals with psoriasis. Psoriasis is a T-cell mediated inflammatory disorder of the skin that can cause considerable discomfort. It is a disease for which there is currently no cure and it affects people of all ages. Although individuals with mild psoriasis can often control their disease with topical agents, more than one million patients worldwide require ultraviolet light treatments or systemic immunosuppressive therapy. Unfortunately, the inconvenience and risks of ultraviolet radiation and the toxicities of many therapies limit their long-term use. Moreover, patients usually have recurrence of psoriasis, and in some cases rebound shortly after stopping immunosuppressive therapy. A recently developed model of psoriasis based on the transfer of CD4+ T cells mimics many aspects of human psoriasis and therefore can be used to identify compounds suitable for use in treatment of psoriasis (Davenport et al., Internat. Immunopharmacol 2:653-672, 2002). Efficacy in this model is a measured by reduction in skin pathology using a scoring system. Similarly, efficacy in patients is measured by a decrease in skin pathology.

TREM-1 antibodies of the invention may be suitable for use in the treatment of individuals with psoriatic arthritis. Psoriatic arthritis (PA) is a type of inflammatory arthritis that occurs in a subset of patients with psoriasis. In these patients, the skin pathology/symptoms are accompanied by a joint swelling similar to that seen in rheumatoid arthritis. It features patchy, raised, red areas of skin inflammation with scaling. Psoriasis often affects the tips of the elbows and knees, the scalp, the navel and around the genital areas or anus. Approximately 10% of patients who have psoriasis also develop an associated inflammation of their joints.

The term "treatment", as used herein, refers to the medical therapy of any human or other animal subject in need thereof. Said subject is expected to have undergone physical examination by a medical or veterinary medical practitioner, who has given a tentative or definitive diagnosis which would indicate that the use of said treatment is beneficial to the health of said human or other animal subject. The timing and purpose of said treatment may vary from one individual to another, according to many factors, such as the status quo of the subject's health. Thus, said treatment may be prophylactic, palliative, symptomatic and/or curative.

In terms of the present invention, prophylactic, palliative, symptomatic and/or curative treatments may represent separate aspects of the invention.

An antibody of the invention may be administered parenterally, such as intravenously, such as intramuscularly, such as subcutaneously. Alternatively, an antibody of the invention may be administered via a non-parenteral route, such as perorally or topically. An antibody of the invention may be administered prophylactically. An antibody of the invention may be administered therapeutically (on demand).

In a first aspect of the invention, the substitution of negatively charged residues of the CDR1 and CDR3 regions of SEQ. ID. NO 3 (the variable light chain of mAb 0170) was observed to influence the viscosity of the mAb. In this first aspect of the invention, the mAb of the invention is a variant of mAb 0170 having a heavy chain and a light chain, wherein the light chain of mAb 0170, namely SEQ. ID. NO 3, comprises mutations wherein negatively charged residues in CDR1 and CDR3 region of SEQ. ID. NO 3 are substituted with uncharged residues. Accordingly, an aspect of the invention is directed to a variant of mAb 0170 comprising substituting any one or any combination of residues D1, D30, D33, D74, D98, E27, E97 of SEQ. ID. NO 3 with an amino acid residue selected from the group consisting of glycine, alanine, serine, asparagine, glutamine, threonine, cysteine, and tyrosine. Otherwise stated, an interesting embodiment of the invention is directed to an antibody or fragment thereof comprising SEQ. ID. NO 2 (or a variant thereof) as a heavy chain and as light chain comprising a variant of SEQ. ID. NO 3 wherein any one or any combination of residues D1, D30, D33, D74, D98, E27, E97 of SEQ. ID. NO 3 is mutated to an amino acid residue selected from the group consisting of glycine, alanine, serine, asparagine, glutamine, threonine, cysteine, and tyrosine. These mutations will be referred to as 'charge patch' mutations. In a preferred embodiment, at least one or both of E27 and E97 of the CDR1 and CDR3 regions of SEQ. ID. NO 3 are substituted with uncharged amino acid residues, such as an amino acid selected from the group consisting of glycine, alanine, serine, asparagine, glutamine, threonine, cysteine, and tyrosine. In a preferred embodiment, E27 of the CDR1 and CDR3 regions of SEQ. ID. NO 3 is mutated to glutamine and E97 is substituted with an amino acid selected from the group consisting of glycine, alanine, serine, asparagine, glutamine, threonine, cysteine, and tyrosine, more preferably with an amino acid selected from the group consisting of serine and glutamine. In a further embodiment, E27 remains unmutated and E97 is mutated with an amino acid selected from the group consisting of glycine, alanine, serine, asparagine, glutamine, threonine, cysteine, and tyrosine, more preferably with an amino acid selected from the group consisting of serine and glutamine. In another embodiment, residue E97 remains unmutated and E27 is substituted with an amino acid selected from the group consisting of glycine, alanine, serine, asparagine, glutamine, threonine, cysteine, and tyrosine, more preferably with an amino acid selected from the group consisting of serine and glutamine.

Another aspect of the invention is based on the observation that Fab-Fab dimers were formed due to interactions in the paratope area. Since mAbs comprise two Fabs, it was envisioned that mAbs would be able to multimerize, which could have an impact on the viscosity properties. Thus, another aspect of the invention is directed to mutate residues in Fab-Fab interaction area of SEQ. ID. NO 2 (namely in the variable heavy chain area of mAb 0170) to reduce Fab-Fab dimerization. These mutations are referred to as 'Fab-Fab interaction' mutations. Accordingly, an aspect of the invention is directed to substituting any one of residues R52, S53, N57, A59, M96, I98 of SEQ. ID. NO 2 or F32, Y30 of SEQ. ID NO 3 with an amino acid residue selected from the group consisting of glycine, alanine, serine, asparagine, glutamine, threonine, cysteine, lysine, arginine, tryptophan, histidine and tyrosine. Otherwise stated, an interesting embodiment of the invention is directed to an antibody or fragment thereof comprising a variant of SEQ. ID. NO 2 wherein any one of residues R52, S53, N57, A59, M96, I98 ('Fab-Fab interaction' mutations) of SEQ. ID. NO 2 or F32, Y30 of SEQ. ID NO 3 is substituted with an amino acid residue selected from the group consisting of glycine, alanine, serine, asparagine, glutamine, threonine, cysteine, lysine, arginine, tryptophan, histidine and tyrosine. Preferably, at least one of residues A59 and N57 SEQ. ID. NO 2 is mutated to an amino acid residue selected from the group consisting of glycine, alanine, serine, asparagine, glutamine, threonine, cysteine, lysine, arginine, tryptophan, histidine and tyrosine, more preferably serine or tyrosine. In one embodiment, A59 remains unmutated and N57 is mutated to an amino acid residue selected from the group consisting of glycine, alanine, serine, asparagine, glutamine, threonine, cysteine, lysine, arginine, tryptophan, histidine and tyrosine, more preferably serine or tyrosine. In another embodiment, N57 remains unmutated and A59 is mutated to an amino acid residue selected from the group consisting of glycine, alanine, serine, asparagine, glutamine, threonine, cysteine, lysine, arginine, tryptophan, histidine and tyrosine. In at least one embodiment, both A59 and N57 are mutated to an amino acid residue selected from the group consisting of glycine, alanine, serine, asparagine, glutamine, threonine, cysteine, lysine, arginine, tryptophan, histidine and tyrosine, more preferably serine or tyrosine.

In an interesting embodiment, i) at least one negatively charged residues of the CDR1 and CDR3 regions of SEQ. ID. NO 3 is mutated to an amino acid residue selected from the group consisting of glycine, alanine, serine, asparagine, glutamine, threonine, cysteine, and tyrosine and

ii) at least one residue of the Fab-Fab interaction area of SEQ. ID. NO 2 ,namely of any one of residues R52, S53, N57, A59, M96, I98 of SEQ. ID. NO 2 or F32, Y30 of SEQ. ID NO 3 is mutated to an amino acid residue selected from the group consisting of glycine, alanine, serine, asparagine, glutamine, threonine, cysteine, lysine, arginine, tryptophan, histidine and tyrosine.

In a suitable embodiment, one or both of E27 and E97 of SEQ. ID. NO 3 is/are mutated to an amino acid residue selected from the group consisting of glycine, alanine, serine, asparagine, glutamine, threonine, cysteine, and tyrosine and one or both of A59 and N57 of SEQ. ID. NO 2 is/are mutated to an amino acid residue selected from the group consisting of glycine, alanine, serine, asparagine, glutamine, threonine, cysteine, lysine, arginine, tryptophan, histidine and tyrosine, more preferably serine or tyrosine.

A further aspect of the invention is based on the observation that an Ala substitution in position Y90 of SEQ. ID NO 1 of TREM-1 improved the affinity of SEQ. ID NO 3 to TREM-1. The Y90 was found to interact with a phenylalanine residue of SEQ. ID. NO 3. Mutation of SEQ. ID. NO 3 in order to improve the Fab-TREM-1 interaction are referred to as Fab-TREM-1 interaction mutations. In one embodiment of this aspect of the invention, phenylalanine at position 32 of SEQ. ID. NO 3 is mutated to an amino acid selected from amino acid residues glycine, serine, threonine, cysteine, alanine, valine, leucine, isoleucine and methionine, preferably selected from alanine, glycine, serine valine and leucine. In an interesting embodiment, F32 of SEQ. ID. NO 3 is mutated to alanine or serine.

Charge patch mutations in the CDR1 and CDR3 of SEQ. ID. NO 3 reduced the hydrodynamic radius (Rh) while mutations in the Fab-Fab interaction region increased Rh. Mutations in the Fab-TREM-1 interaction site did not influence Rh.

Table 1: Overview of SEQ ID No (0170) mAb variants generated. LC = light chain, HC = heavy chain.

| | | mAb ID | Comprise Sequence ID No. | Mutation(s) to SEQ. ID. NO 2 or 3 | Chain |
|---|---|---|---|---|---|
| **Charge patch mutations** | | 0317 | SEQ. ID. NO 4 and 2 | E27Q, E97S | LC |
| | | 0318 | SEQ. ID. NO 5 and 2 | E27Q, E97Q | LC |
| | | 0319 | SEQ. ID. NO 6 and 2 | E97S | LC |
| | | 0320 | SEQ. ID. NO 7 and 2 | E97Q | LC |
| | | 0321 | SEQ. ID. NO 8 and 2 | E27Q | LC |

(continued)

|  | mAb ID | Comprise Sequence ID No. | Mutation(s) to SEQ. ID. NO 2 or 3 | Chain |
|---|---|---|---|---|
| **Fab-Fab interaction mutations** | 0324 | SEQ. ID. NO 11 and 3 | A59Y | HC |
|  | 0325 | SEQ. ID. NO 12 and 3 | N57S | HC |
|  | 0326 | SEQ. ID. NO 13 and 3 | A59Y,N57S | HC |
| **Fab-TREM-1 interaction mutations** | 0322 | SEQ. ID. NO 9 and 2 | F32A | LC |
|  | 0323 | SEQ. ID. NO 10 and 2 | F32S | LC |

The viscosity was determined for the mAb 0170 variants by DLS and showed that both charge patch mutations and Fab-Fab interaction mutations reduced the viscosity. The mAb variant 0318, which comprise the charge patch mutations E27Q and E97Q was found to have the lowest viscosity.

The binding kinetics of the mAb 0170 variants towards human TREM-1-Fc and cynomolgus TREM-1-Fc were determined, respectively. The mAb 0170 variants were all found to have similar affinity towards human TREM-1-Fc. Interestingly, the mAb variant, SEQ. ID. NO 9 comprising a Fab-TREM-1 mutation was found to have increased affinity towards cynomolgus TREM-1-Fc.

Table 2: Affinity of anti-TREM-1 mAb variants towards human TREM-1-Fc and cynomolgus TREM-1-Fc respectively.

| **Human TREM-1-Fc** | | | | **Cynomolgus TREM-1-Fc** | | | |
|---|---|---|---|---|---|---|---|
| mAb | ka (1/Ms) | kd (1/s) | KD (nM) | ka (1/Ms) | kd (1/s) | KD (nM) |
| 0170 | 3E+06 | 5E-04 | 0.1 | 0.3E+06 | 4E-04 | 2 |
| 0317 | 4E+06 | 6E-04 | 0.2 | 0.3E+06 | 7E-04 | 3 |
| 0318 | 4E+06 | 8E-04 | 0.2 | 0.3E+06 | 7E-04 | 2 |
| 0319 | 4E+06 | 6E-04 | 0.2 | 0.3E+06 | 6E-04 | 2 |
| 0320 | 4E+06 | 7E-04 | 0.2 | 0.2E+06 | 6E-04 | 3 |
| 0321 | 3E+06 | 6E-04 | 0.2 | 0.3E+06 | 6E-04 | 2 |
| 0322 | 3E+06 | 3E-04 | 0.1 | 0.5E+06 | 1E-04 | 0.2 |
| 0323 | 2E+06 | 6E-04 | 0.3 | 0.4E+06 | 2E-04 | 0.6 |
| 0324 | 3E+06 | 8E-04 | 0.3 | 0.2E+06 | 6E-04 | 4 |
| 0325 | 4E+06 | 3E-04 | 0.08 | 0.2E+06 | 6E-04 | 1 |
| 0326 | 3E+06 | 7E-04 | 0.2 | 0.2E+06 | 3E-04 | 5 |
| 0330 | 2E+06 | 3E-04 | 0.1 | 6E+06 | 0.9E-04 | 0.1 |

In an embodiment with both improved affinity towards TREM-1 and having low viscosity, the mutations from SEQ. ID. NO 9 were combined with the mutations in SEQ. ID. NO 5. The increased affinity towards cynomolgus TREM-1-Fc was retained for the mAb variant comprising the combined mutations of SEQ. ID. NO 14 and the mutations did not negatively affect the affinity towards human TREM-1-Fc. Accordingly, embodiments comprising both charge patch mutations and Fab-TREM-1 interaction mutations are envisaged by the present invention, as well as embodiments comprising both charge patch mutations and Fab-Fab interaction mutations as well as embodiments comprising both Fab-Fab interaction mutations and Fab-TREM-1 interaction mutations.

Table 3A: Viscosity versus protein concentrations of mAb 0170, 0317 and 0318

| mAb | | | | | |
|---|---|---|---|---|---|
| **0170** | | **0317** | | **0318** | |
| Protein concentration (mg/mL) | Viscosity (cP) | Protein concentration (mg/mL) | Viscosity (cP) | Protein concentration (mg/mL) | Viscosity (cP) |
| 100.0 | 26.0 | 85.0 | 2.9 | 85.0 | 1.9 |
| 80.0 | 14.5 | 63.8 | 2.2 | 63.8 | 1.9 |

(continued)

| mAb | | | | | |
|---|---|---|---|---|---|
| **0170** | | **0317** | | **0318** | |
| Protein concentration (mg/mL) | Viscosity (cP) | Protein concentration (mg/mL) | Viscosity (cP) | Protein concentration (mg/mL) | Viscosity (cP) |
| 60.0 | 6.0 | 42.5 | 1.9 | 42.5 | 2.0 |
| 40.0 | 3.1 | 17.0 | 1.3 | 17.0 | 1.2 |
| 20.0 | 1.6 | 10.2 | 1.2 | 10.2 | 1.2 |
| 12.0 | 1.3 | 3.4 | 1.1 | 3.4 | 1.2 |
| 4.0 | 1.1 | 0.0 | 1.0 | 0.0 | 1.0 |
| 0.0 | 0.9 | | | | |

Table 3B: Viscosity versus protein concentrations of mAb 0319 and 0320 and 321.

| mAb | | | | | |
|---|---|---|---|---|---|
| **0319** | | **0320** | | **0321** | |
| Protein concentration (mg/mL) | Viscosity (cP) | Protein concentration (mg/mL) | Viscosity (cP) | Protein concentration (mg/mL) | Viscosity (cP) |
| 85.0 | 2.2 | 107.0 | 7.8 | 106.0 | 5.9 |
| 63.8 | 2.3 | 80.3 | 3.7 | 79.5 | 3.0 |
| 42.5 | 2.0 | 53.5 | 2.5 | 53.0 | 2.0 |
| 17.0 | 1.2 | 26.8 | 1.6 | 26.5 | 1.6 |
| 10.2 | 1.1 | 13.4 | 1.3 | 13.3 | 1.2 |
| 3.4 | 1.1 | 6.7 | 1.2 | 6.6 | 1.1 |
| 0.0 | 1.0 | 3.3 | 1.1 | 3.3 | 1.1 |
| | | 0.0 | 1.0 | 0.0 | 0.9 |

Table 3C: Viscosity versus protein concentrations of mAb 0322, 0323 and 324.

| mAb | | | | | |
|---|---|---|---|---|---|
| **0322** | | **0323** | | **0324** | |
| Protein concentration (mg/mL) | Viscosity (cP) | Protein concentration (mg/mL) | Viscosity (cP) | Protein concentration (mg/mL) | Viscosity (cP) |
| 100.0 | 16.4 | 101.0 | 21.3 | 103.0 | 5.4 |
| 75.0 | 4.9 | 75.8 | 11.3 | 77.3 | 3.1 |
| 50.0 | 3.3 | 50.5 | 3.7 | 51.5 | 2.2 |
| 25.0 | 2.0 | 25.3 | 2.6 | 25.8 | 1.6 |
| 12.5 | 1.6 | 12.6 | 2.0 | 12.9 | 1.4 |
| 6.3 | 1.4 | 6.3 | 1.4 | 6.4 | 1.3 |
| 3.1 | 1.3 | 3.2 | 1.2 | 3.2 | 1.2 |
| 0.0 | 1.0 | 0.0 | 1.0 | 0.0 | 0.9 |

Table 3C: Viscosity versus protein concentrations of mAb 0325 and 326.

| mAb | | | |
|---|---|---|---|
| **0325** | | **0326** | |
| Protein concentration (mg/mL) | Viscosity (cP) | Protein concentration (mg/mL) | Viscosity (cP) |
| 95.0 | 7.8 | 109.0 | 7.5 |
| 71.3 | 3.7 | 81.8 | 3.1 |
| 47.5 | 2.3 | 54.5 | 2.2 |
| 23.8 | 1.6 | 27.3 | 1.6 |
| 11.9 | 1.3 | 6.8 | 1.5 |
| 5.9 | 1.3 | 3.4 | 1.1 |
| 3.0 | 1.2 | 0.0 | 0.9 |
| 0.0 | 1.0 | | |

Non-limiting embodiments of the present invention further comprise:

1. An antibody or fragment thereof that is capable of binding to and blocking TREM-1 of SEQ. ID. NO: 1, characterized in that the antibody or an antibody fragment of said antibody has a viscosity of less than 5 cP at a concentration of 50 mg/mL, such as less than 4 cP, preferably less than 3 cP (wherein the method of determining viscosity versus protein concentration conditions are conventional or as described herein.)

2. An antibody or fragment thereof that is capable of binding to and blocking TREM-1, characterized in that the antibody or an antibody fragment of said antibody, has a viscosity of less than 5 cP at a concentration of 80 mg/mL, preferably less than 4 cP. (wherein the method of determining viscosity versus protein concentration conditions are conventional or as described herein.)

3. An antibody or fragment thereof according to embodiments 1 or 2 wherein the antibody or fragment thereof blocks TREM-1 function with a KD to SEQ. ID. NO: 1 of less than 0.5 nM, such as less than 0.4 nM, preferably of 0.3 nM or less.

4. An antibody or fragment thereof wherein the antibody or fragment thereof competitively binds with mAb 0170 for binding to SEQ ID NO: 1 and has a viscosity profile such that:

a. the antibody or an antibody fragment of said antibody has a viscosity of less than 5 cP at a concentration of 50 mg/mL, such as less than 4 cP, preferably less than 3 cP ; or
b. the antibody or an antibody fragment of said antibody, has a viscosity of less than 5 cP at a concentration of 80 mg/mL, preferably less than 4 cP.

5. An antibody or fragment thereof that is capable of specifically binding to and blocking TREM-1, wherein the antibody or fragment thereof has 'Fab-Fab interaction' mutations of SEQ. ID. NO 2.

6. An antibody or fragment thereof that is capable of specifically binding to and blocking TREM-1 of SEQ. ID. NO 1, wherein the antibody or fragment thereof has 'charge patch' mutations, such as wherein at least one of the negatively charged residues of the CDR1 and CDR3 of SEQ. ID. NO 3 are substituted with uncharged residues.

7. An antibody or fragment thereof according to any of the embodiments of the invention wherein negatively charged amino acids involved in the protein-protein interaction are substituted with amino acid residues which can form hydrogen bonding partners, such as mutation of an Asp or Glu residue to any one of Asn, Gin, Ser and Thr.

8. An antibody or fragment thereof according to any of the embodiments of the invention having a lowered the viscosity compared to mAb 0170, but having a KD to SEQ. ID. NO: 1 of less than 0.5 nM, by substituting uncharged amino acids of SEQ. ID. NO 2 and/or 3, involved in specific mAb-mAb self-interactions with amino acids with altered size or hydrogen bonding potential.

9. An antibody or fragment thereof according to any of the embodiments of the invention that is capable of specifically binding to and blocking TREM-1 of SEQ. ID. NO: 1 and comprises variants of SEQ. ID. NO: 2 or SEQ. ID. NO: 3 or both, wherein the variants are selected from the group consisting of 'Fab-Fab interaction' mutations, 'Fab-TREM-1 interaction' mutations and 'charge-patch" mutations of SEQ. ID. NO: 2 or SEQ. ID. NO: 3.

10. An antibody or fragment thereof according to any of the embodiments of the invention comprising 'Fab-TREM-1 interaction' mutations, such as wherein
a Phe residue of SEQ. ID. NO: 2 is substituted with Ala or Ser.

11. An antibody or fragment thereof according to any of the embodiments of the invention which competes with SEQ. ID. NO: 2 or 3 for binding to SEQ ID NO: 1 and which has an epitope comprising one, two, three, four, five, six, seven or all of the amino acid residues D38, V39, K40, C41, D42, Y43, T44 and L45 and one, two or all of the amino acid residues E46, K47, F48 of SEQ ID NO: 1.

12. The antibody or fragment thereof according to any of the embodiments of the invention, which competes for binding with SEQ ID NO: 3, and comprising a LC having one or both glutamate (E) residues of positions 27 and 97 of SEQ ID NO: 3 mutated to serine (S) or glutamine (Q), such as wherein E27Q, E97S, such as wherein both E27 and E97 are mutated to glutamine or wherein E27 remains un-mutated and E97 is mutated to S (E27, E97S), or wherein E27 remains un-mutated and E97 is mutated to Q (E27, E97Q), or wherein E97 remains un-mutated and E27 is mutated to Q or S, more preferably to Q (E27, E97Q).

13. The antibody or fragment thereof according to any of the embodiments of the invention comprising SEQ ID NO: 3, wherein the phenylalanine at position 32 (F32) is mutated, such as mutated to A (0322) or to S (0323).

14. The antibody or fragment thereof according to any one of embodiments, comprising SEQ ID NO: 2 wherein one or both of residue 57 and residue 59 has been mutated, such as wherein residue 59 has been mutated to a tyrosine, or wherein residue 57 has been mutated to a serine, or wherein residue 57 has been mutated to a serine and residue 59 has been mutated to a tyrosine.

15. An antibody or fragment thereof comprising a variant of SEQ. ID. NO 2 or a variant of SEQ. ID. NO 3, wherein the VH or VL regions are mutated wherein uncharged amino acids involved in specific mAb-mAb self-interactions are substituted with amino acids with altered size or hydrogen bonding potential, such as wherein Asp or Glu are substituted with any one of Asn, Gln, Ser and Thr.

16. An antibody or fragment thereof comprising a variant of SEQ ID NO: 3 and which competes for binding with SEQ ID NO: 3 wherein one or both glutamate (E) residues of positions 27 and 97 of SEQ ID NO: 3 are mutated to serine (S) or glutamine (Q), such as wherein E27Q, E97S, such as wherein both E27 and E97 are mutated to glutamine (318), or wherein E27 remains un-mutated and E97 is mutated to S (E27, E97S), or wherein E27 remains un-mutated and E97 is mutated to Q (E27, E97Q), or wherein E97 remains un-mutated and E27 is mutated to Q or S, more preferably to Q (E27, E97Q).

17. An antibody or fragment thereof comprising a variant of SEQ ID NO: 2 and which competes for binding with SEQ ID NO: 2, wherein one or both of residue 57 and residue 59 has been mutated, such as wherein residue 59 has been mutated to a tyrosine, or wherein residue 57 has been mutated to a serine, or wherein both residue 57 has been mutated to a serine and residue 59 has been mutated to a tyrosine.

18. An antibody or fragment thereof comprising SEQ ID NO: 4.

19. An antibody or fragment thereof comprising SEQ ID NO: 5.

20. An antibody or fragment thereof comprising SEQ ID NO: 6.

21. An antibody or fragment thereof comprising SEQ ID NO: 7.

22. An antibody or fragment thereof comprising SEQ ID NO: 8.

23. An antibody or fragment thereof comprising SEQ ID NO: 9.

24. An antibody or fragment thereof comprising SEQ ID NO: 10.

25. An antibody or fragment thereof comprising SEQ ID NO: 11.

26. An antibody or fragment thereof comprising SEQ ID NO: 12.

27. An antibody or fragment thereof comprising SEQ ID NO: 13.

28. An antibody or fragment thereof comprising SEQ ID NO: 14.

29. An antibody or fragment thereof comprising SEQ ID NO: 15.

30. An antibody or fragment thereof comprising SEQ ID NO: 16.

31. An antibody or fragment thereof comprising any one of SEQ ID NOs: 4 to 16.

32. A composition comprising an antibody as defined in embodiment 29.

33. An antibody of the invention, such as of embodiment 29, for use as a medicament.

34. An antibody of the invention, such as of embodiment 29, for use in the treatment of a disease selected from an inflammatory disease or an autoimmune disease.

35. An antibody according to embodiment 32, wherein the disease is selected from the group consisting of rheumatoid arthritis and inflammatory bowel disease.

36. Use of an antibody of the invention, such as of embodiment 29, for the preparation of a medicament for the treatment of a disease selected from the group consisting of an inflammatory disease or an autoimmune disease.

37. The use according to embodiment 34 wherein the disease is selected from the group consisting of rheumatoid arthritis and inflammatory bowel disease.

38. A method of treating a disease selected from an inflammatory disease or an autoimmune disease comprising administering an antibody of the invention, a composition comprising the antibody or a medicament comprising the antibody of the invention.

**EXAMPLES**

Example 1: Hydrodynamic radius and viscosity of mAb0170 variants

The hydrodynamic radius of mAb0170 of WO2013/120553 was found to be higher (9-10 nm) than desired (5-6 nm). The hydrodynamic radius was determined by dynamic light scattering analysis using a 96-well plate setup with a DynaPro plate reader (Wyatt Inc.). The plates used were Corning 3540 assay plates (Corning). Total sample volume was approximately 20 $\mu$L and the temperature was kept at 25 °C for the mAb0170 variants (Figure 7).

Charge patch mutations in the CDR1 and CDR3 of the variable light chain reduced the Rh to the level expected for monomeric mAbs while mutations in the Fab-Fab interaction region seemed to increase Rh. Mutations in the Fab-TREM-1 interaction site did not influence Rh.

The viscosity was determined for the mAb0170 variants by DLS. Hydrodynamic radius was measured on a Wyatt DynaPro Platereader using Corning 3540 clear bottom, black not-treated polystyrene microplates and plain polystyrene nanospheres from Phosphorex Inc. with a mean diameter of 206.5 nm (Cat. no. 106). The protein samples were transferred to a Corning 3540 plate and covered with a top seal. The samples were centrifuged and the hydrodynamic radius of the protein samples were measured in the Wyatt DynaPro DLS plate reader. Each well was added 0.5 $\mu$L polystyrene beads and mixed gently by pipetting. The plate was centrifuged again and the hydrodynamic radii of the beads were measured in the Wyatt DynaPro DLS plate reader.

The viscosities of the protein samples were calculated as

$$viscosity(protein) = (hydrodynamic\ radius(beads,meas) \times viscosity(buffer))/hydrodynamic$$
$$radius(beads,\ real)$$

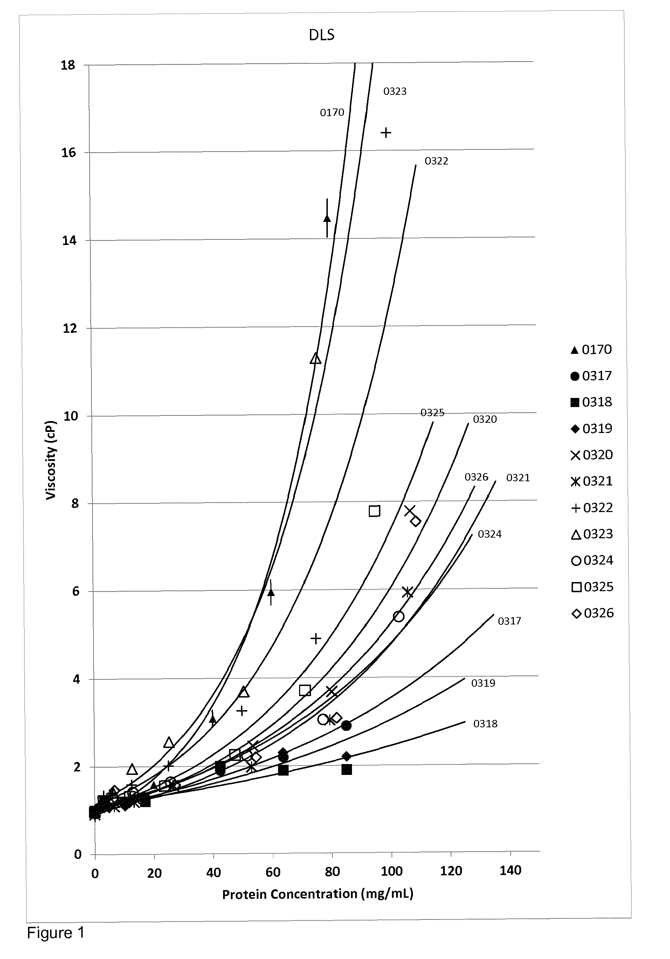

where viscosity(protein) is the calculated viscosity of the protein solution, hydrodynamic radius(beads, meas) is the measured hydrodynamic radius of the polystyrene beads in the protein solution, viscosity(buffer) is the viscosity of the buffer and hydrodynamic radius(beads, real) is the real mean diameter of the beads [He et al., Anal Biochem, 399, 141-143, 2010]. The analysis showed that both charge patch mutations and Fab-Fab interaction mutations reduced the viscosity (Figure 1). The mAb variant SEQ. ID. No. 5, which comprises the charge patch mutations E27Q and E97Q was found to have the lowest viscosity.

Example 2: Kinetics of mAb0170 variants

The binding kinetics of the mAb0170 variants towards human TREM-1-Fc and cynomolgus TREM-1-Fc were determined, respectively. The NNC0254-0000-0170 mAb variants were all found to have similar affinity towards human TREM-1-Fc as mAb0170 variants. Interestingly, mAb variants comprising a Fab-TREM-1 mutation, such as SEQ ID No:9, had increased affinity towards cynomolgus TREM-1-Fc (Table 2).

Example 3: Kinetics and viscosity of SEQ ID NO 14

In the attempt to generate a mAb with both improved affinity towards cynomolgus TREM-1 and having low viscosity, the mutations from SEQ ID NO 9 was combined with the mutations in SEQ ID NO 5, which was found to have the lowest viscosity among the mAb0170 variants. The increased affinity towards cynomolgus TREM-1-Fc was retained for the mAb variant comprising the combined mutations (SEQ ID NO 14) and the mutations did not affect the affinity towards human TREM-1-Fc. The viscosity of SEQ ID NO 14 was markedly reduced compared to mAB0170 of WO2013/120553 (Table 2).

Example 4: Functional characterization mAb0170 variants

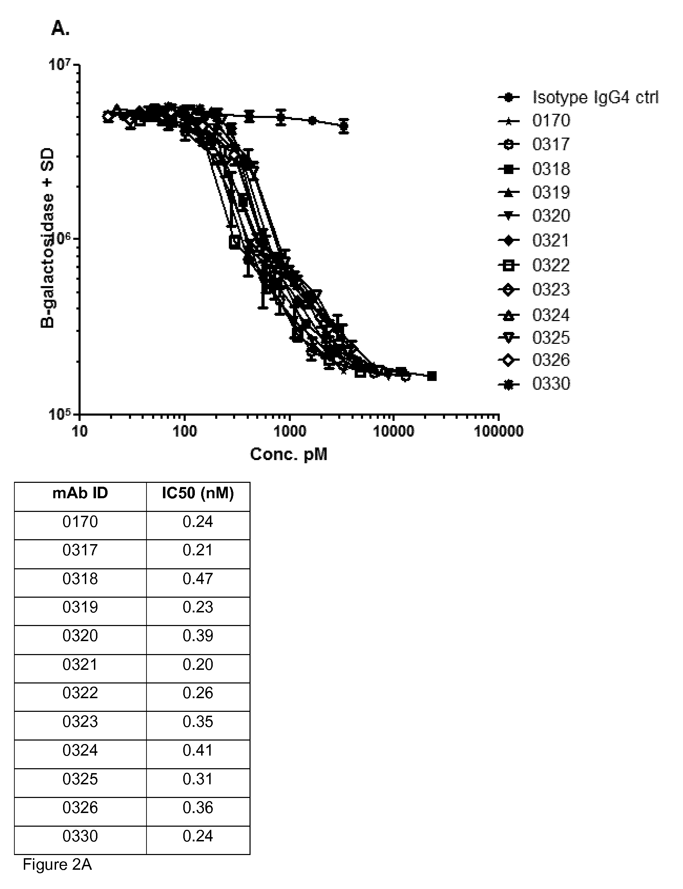

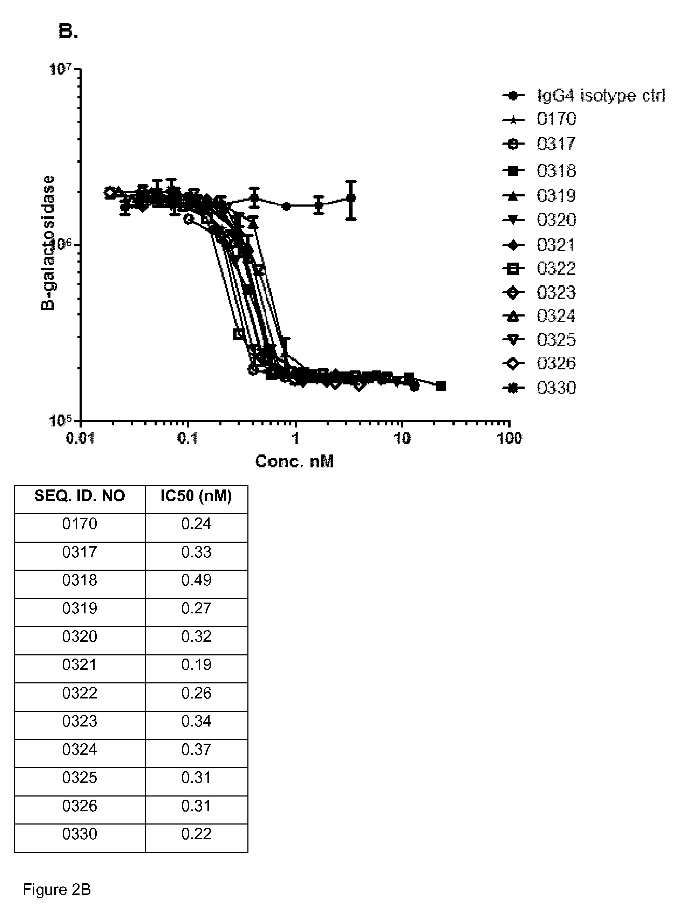

The ability of the anti-TREM-1 mAb0170 variants to inhibit human TREM-1 signalling was determined using a reporter cell line (BWZ'36/hTREM-1) provided by Bioxell. The reporter cell line was stimulated with PGN and TREM-1 ligand PGLYRP1 either as recombinant protein or expressed by activated neutrophils prior to incubation with mAbs. The potency of the variants to inhibit TREM-1 signalling was found to be clinical relevance and on par with that of the mAb0170 of WO2013/120553 (Figure 2A and B).

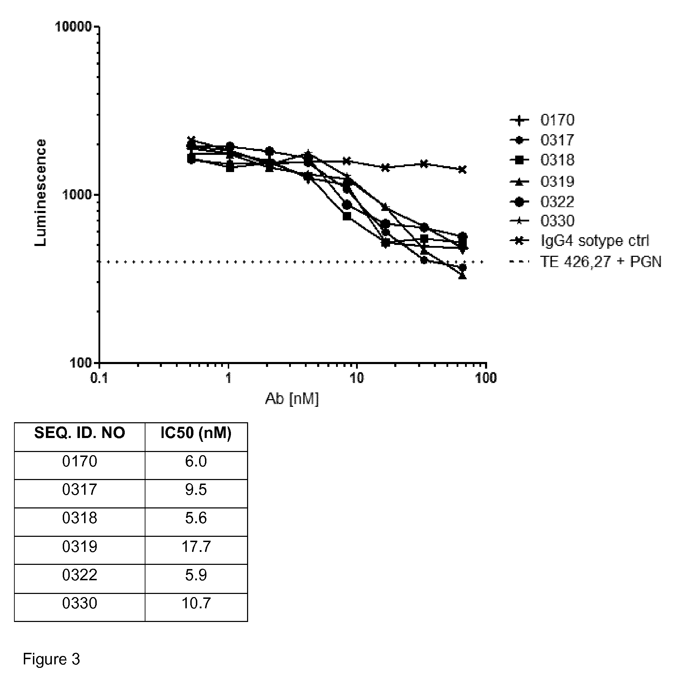

Likewise, the ability to inhibit cynomolgus TREM-1 signalling was determined for the three mAb0170 variants with the lowest viscosity and the two variants observed to have increased affinity towards cynomolgus TREM-1. In this assay using an established reporter cell line TE 426.27, similar potencies of the mAb variants and mAb0170 of WO2013/120553 was observed (Figure 3).

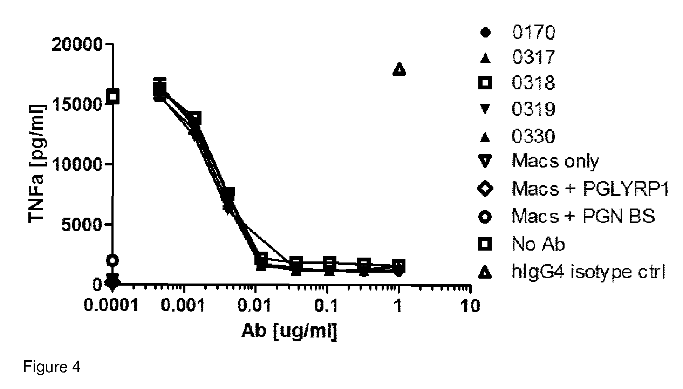

Also, the potency of the mAb variants to block TNFa release from primary cells from healthy donors was assessed. In this assay monocytes were differentiated to M2 macrophages under hypoxic conditions in order to increase TREM-1 expression and activated with PGN and recombinant PGLYRP1 prior to incubation with mAb. The mAb variants were found to be as potent as 0170 at inhibiting TREM-1-mediated TNFa release from hypoxic M2 macrophages (Figure 4).

## Claims

1. An antibody or fragment thereof that is capable of binding to and blocking TREM-1, **characterized in that** the antibody or an antibody fragment of said antibody has a viscosity of less than 5 cP at a concentration of 80 mg/mL, preferably less than 4 cP.

2. An antibody or fragment thereof wherein one or more of the negatively charged residues in CDR1 and CDR3 region of SEQ. ID. NO 3 are substituted with uncharged amino acid residues.

3. An antibody or fragment thereof according to any of the preceding claims, comprising a variant of SEQ. ID. NO 3 wherein any one or more of residues D1, D27, D29, D70, D94, E27, E97 ('charge patch' mutations) of SEQ. ID. NO 3 is mutated to an amino acid residue selected from the group consisting of glycine, alanine, serine, asparagine, glutamine, threonine, cysteine, and tyrosine.

**4.** An antibody or fragment thereof according to claim 3 wherein at least one or both of E27 and E97 of the CDR1 and CDR3 regions of SEQ. ID. NO 3 are substituted with uncharged amino acid residues, such as an amino acid selected from the group consisting of glycine, alanine, serine, asparagine, glutamine, threonine, cysteine, and tyrosine.

**5.** An antibody or fragment thereof according to any one of claims 3 to 4, wherein E27 of SEQ. ID. NO 3 is mutated to glutamine and E97 is substituted with an amino acid selected from the group consisting of glycine, serine, asparagine, glutamine, threonine, cysteine, and tyrosine, more preferably with an amino acid selected from the group consisting of serine and glutamine.

**6.** An antibody or fragment thereof according to any one of claims 3 to 4, wherein E27 of SEQ. ID. NO 3 remains unmutated and E97 is mutated with an amino acid selected from the group consisting of glycine, alanine, serine, asparagine, glutamine, threonine, cysteine, and tyrosine, more preferably with an amino acid selected from the group consisting of serine and glutamine.

**7.** An antibody or fragment thereof according to any one of claims 3 to 4, wherein residue E97 of SEQ. ID. NO 3 remains unmutated and E27 is substituted with an amino acid selected from the group consisting of glycine, alanine, serine, asparagine, glutamine, threonine, cysteine, and tyrosine, more preferably with an amino acid selected from the group consisting of serine and glutamine.

**8.** An antibody or fragment thereof comprising a variant of SEQ. ID. NO 2 wherein any one of residues R52, S53, N57, A59, M96, I98 of SEQ. ID. NO 2 or F32, Y30 of SEQ. ID NO 3 ('Fab-Fab interaction' mutations) is substituted with another amino acid, such as natural amino acid, preferably an amino acid residue selected from the group consisting of glycine, alanine, serine, asparagine, glutamine, threonine, cysteine, lysine, arginine, tryptophan, histidine and tyrosine.

**9.** An antibody or fragment thereof according to claim 8, wherein at least one of residues A59 and N57 of SEQ. ID. NO 2 is mutated to an amino acid residue, such as a natural amino acid, preferably an amino acid selected from the group consisting of glycine, alanine, serine, asparagine, glutamine, threonine, cysteine, lysine, arginine, tryptophan, histidine and tyrosine, more preferably serine or tyrosine.

**10.** An antibody or fragment thereof according to claim 8, wherein A59 of SEQ. ID. NO 2 remains unmutated and N57 is mutated to another amino acid residue, such as natural amino acid, preferably selected from the group consisting of glycine, alanine, serine, asparagine, glutamine, threonine, cysteine, lysine, arginine, tryptophan, histidine and tyrosine, more preferably serine or tyrosine.

**11.** An antibody or fragment thereof according to claim 8, wherein N57 of SEQ. ID. NO 2 remains unmutated and A59 of SEQ. ID. NO 2 is mutated to another amino acid residue, such as natural amino acid, preferably selected from the group consisting of glycine, alanine, serine, asparagine, glutamine, threonine, cysteine, lysine, arginine, tryptophan, histidine and tyrosine, more preferably serine or tyrosine.

**12.** An antibody or fragment thereof according to claim 8, wherein both A59 and N57 of SEQ. ID. NO 2 are mutated to another amino acid residue, such as natural amino acid, preferably selected from the group consisting glycine, alanine, serine, asparagine, glutamine, threonine, cysteine, lysine, arginine, tryptophan, histidine and tyrosine, more preferably serine or tyrosine.

**13.** An antibody or fragment thereof according to any one of the preceding claims, wherein

i) at least one negatively charged residues of the CDR1 and CDR3 regions of SEQ. ID. NO 3 is mutated to an amino acid residue selected from the group consisting of glycine, alanine, serine, asparagine, glutamine, threonine, cysteine, and tyrosine and
ii) at least one residue of residues R52, S53, N57, A59, M96, I98 of SEQ. ID. NO 2 or F32, Y30 of SEQ. ID NO 3 ('Fab-Fab interaction' mutations) is mutated to an amino acid residue selected from the group consisting of glycine, alanine, serine, asparagine, glutamine, threonine, cysteine, lysine, arginine, and tryptophan, histidine and tyrosine.

**14.** An antibody or fragment thereof according to any one of the preceding claims,

wherein one or both of E27 and E97 of SEQ. ID. NO 3 is/are mutated to an amino acid residue selected from the group consisting of glycine, alanine, serine, asparagine, glutamine, threonine, cysteine, and tyrosine; and one or both of A59 and N57 of SEQ. ID. NO 2 is/are mutated to an amino acid residue selected from the group consisting of glycine, alanine, serine, asparagine, glutamine, threonine, cysteine, lysine, arginine, tryptophan, histidine and tyrosine, more preferably serine or tyrosine.

15. An antibody or fragment thereof comprising a variant of SEQ. ID. NO 3 wherein phenylalanine at position 32 of SEQ. ID. NO 3 is mutated to an amino acid selected from amino acid residues glycine, serine, threonine, cysteine, alanine, valine, leucine, isoleucine and methionine, preferably selected from alanine, glycine, serine valine and leucine, more preferably wherein F32 of SEQ. ID. NO 3 is mutated to alanine or serine.

16. An antibody or fragment thereof comprising any one of SEQ ID NOs: 4 to 16.

Figure 1

**A.**

| mAb ID | IC50 (nM) |
|--------|-----------|
| 0170 | 0.24 |
| 0317 | 0.21 |
| 0318 | 0.47 |
| 0319 | 0.23 |
| 0320 | 0.39 |
| 0321 | 0.20 |
| 0322 | 0.26 |
| 0323 | 0.35 |
| 0324 | 0.41 |
| 0325 | 0.31 |
| 0326 | 0.36 |
| 0330 | 0.24 |

Figure 2A

**B.**

| SEQ. ID. NO | IC50 (nM) |
|-------------|-----------|
| 0170 | 0.24 |
| 0317 | 0.33 |
| 0318 | 0.49 |
| 0319 | 0.27 |
| 0320 | 0.32 |
| 0321 | 0.19 |
| 0322 | 0.26 |
| 0323 | 0.34 |
| 0324 | 0.37 |
| 0325 | 0.31 |
| 0326 | 0.31 |
| 0330 | 0.22 |

Figure 2B

| SEQ. ID. NO | IC50 (nM) |
|:-----------:|:---------:|
| 0170 | 6.0 |
| 0317 | 9.5 |
| 0318 | 5.6 |
| 0319 | 17.7 |
| 0322 | 5.9 |
| 0330 | 10.7 |

Figure 3

Figure 4

Figure 5

Figure 6

Europäisches
Patentamt

European
Patent Office

Office européen
des brevets

# EUROPEAN SEARCH REPORT

Application Number

EP 14 17 7547

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2013/120553 A1 (NOVO NORDISK AS [DK]) 22 August 2013 (2013-08-22) * abstract * | 1-16 | INV. C07K16/28 |
| X | WO 2014/072876 A1 (PFIZER [US]) 15 May 2014 (2014-05-15) * page 157; example 10 * | 1-16 | |
| Y | KETCHEM RANDAL R ET AL: "Mitigation of monoclonal antibody viscosity by modification of protein surface charge", AMERICAN CHEMICAL SOCIETY. ABSTRACTS OF PAPERS (AT THE NATIONAL MEETING), AMERICAN CHEMICAL SOCIETY, US , vol. 243 25 March 2012 (2012-03-25), page 1, XP008171180, ISSN: 0065-7727 Retrieved from the Internet: URL:http://abstracts.acs.org/chem/243nm/program/view.php?obj_id=122153&terms= [retrieved on 2015-01-12] * abstract * | 1-16 | |
| Y | ANUJ CHAUDHRI ET AL: "The Role of Amino Acid Sequence in the Self-Association of Therapeutic Monoclonal Antibodies: Insights from Coarse-Grained Modeling", THE JOURNAL OF PHYSICAL CHEMISTRY B, vol. 117, no. 5, 7 February 2013 (2013-02-07), pages 1269-1279, XP055160590, ISSN: 1520-6106, DOI: 10.1021/jp3108396 * abstract * | 1-16 | TECHNICAL FIELDS SEARCHED (IPC) C07K |

-/--

The present search report has been drawn up for all claims

| Place of search | Date of completion of the search | Examiner |
|---|---|---|
| Munich | 15 January 2015 | Lechner, Oskar |

EPO FORM 1503 03.82 (P04C01)

Europäisches
Patentamt

European
Patent Office

Office européen
des brevets

# EUROPEAN SEARCH REPORT

Application Number

EP 14 17 7547

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | SANDEEP YADAV ET AL: "The Influence of Charge Distribution on Self-Association and Viscosity Behavior of Monoclonal Antibody Solutions", MOLECULAR PHARMACEUTICS, vol. 9, no. 4, 2 April 2012 (2012-04-02), pages 791-802, XP055134188, ISSN: 1543-8384, DOI: 10.1021/mp200566k * abstract * | 1-16 | |
| Y | SANDEEP YADAV ET AL: "Establishing a Link Between Amino Acid Sequences and Self-Associating and Viscoelastic Behavior of Two Closely Related Monoclonal Antibodies", PHARMACEUTICAL RESEARCH, KLUWER ACADEMIC PUBLISHERS-PLENUM PUBLISHERS, NL, vol. 28, no. 7, 6 April 2011 (2011-04-06), pages 1750-1764, XP019912566, ISSN: 1573-904X, DOI: 10.1007/S11095-011-0410-0 * abstract * | 1-16 | |
| A | D. BETHEA ET AL: "Mechanisms of self-association of a human monoclonal antibody CNTO607", PROTEIN ENGINEERING DESIGN AND SELECTION, vol. 25, no. 10, 22 August 2012 (2012-08-22), pages 531-538, XP055078364, ISSN: 1741-0126, DOI: 10.1093/protein/gzs047 * abstract * | 1-16 | |

TECHNICAL FIELDS
SEARCHED (IPC)

-/--

The present search report has been drawn up for all claims

| Place of search | Date of completion of the search | Examiner |
|---|---|---|
| Munich | 15 January 2015 | Lechner, Oskar |

EPO FORM 1503 03.82 (P04C01)

Europäisches
Patentamt

European
Patent Office

Office européen
des brevets

# EUROPEAN SEARCH REPORT

Application Number

EP 14 17 7547

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | TIM J KAMERZELL ET AL: "Protein excipient interactions: Mechanisms and biophysical characterization applied to protein formulation development", ADVANCED DRUG DELIVERY REVIEWS, ELSEVIER, AMSTERDAM, NL, vol. 63, no. 13, 26 July 2011 (2011-07-26), pages 1118-1159, XP028320148, ISSN: 0169-409X, DOI: 10.1016/J.ADDR.2011.07.006 [retrieved on 2011-07-29] * the whole document * | 1-16 | |
| A | JAN JEZEK ET AL: "Viscosity of concentrated therapeutic protein compositions", ADVANCED DRUG DELIVERY REVIEWS, ELSEVIER, AMSTERDAM, NL, vol. 63, no. 13, 2 September 2011 (2011-09-02), pages 1107-1117, XP028320147, ISSN: 0169-409X, DOI: 10.1016/J.ADDR.2011.09.008 [retrieved on 2011-10-19] * the whole document * | 1-16 | |
| T | ANJA DRESCHER: "Characterization of biological interactions with Biacore", INTERNET CITATION, 27 June 2011 (2011-06-27), pages 1-26, XP007919211, [retrieved on 2011-08-11] * the whole document * | 1-16 | TECHNICAL FIELDS SEARCHED (IPC) |

The present search report has been drawn up for all claims

| Place of search | Date of completion of the search | Examiner |
|---|---|---|
| Munich | 15 January 2015 | Lechner, Oskar |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 14 17 7547

This annex lists the patent family members relating to the patent documents cited in the above-mentioned European search report.
The members are as contained in the European Patent Office EDP file on
The European Patent Office is in no way liable for these particulars which are merely given for the purpose of information.

15-01-2015

| Patent document cited in search report | Publication date | Patent family member(s) | Publication date |
|---|---|---|---|
| WO 2013120553 A1 | 22-08-2013 | CN 104220456 A<br>EP 2814844 A1<br>US 2013211050 A1<br>WO 2013120553 A1 | 17-12-2014<br>24-12-2014<br>15-08-2013<br>22-08-2013 |
| WO 2014072876 A1 | 15-05-2014 | TW 201431878 A<br>WO 2014072876 A1 | 16-08-2014<br>15-05-2014 |

EPO FORM P0459

For more details about this annex : see Official Journal of the European Patent Office, No. 12/82

**EP 2 975 056 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2013120553 A
- WO 2005040219 A
- US 20050238646 A
- US 20020161201 A
- US 5677425 A, Bodmer

### Non-patent literature cited in the description

- **YDAV et al.** *Mol. Pharmaceutics,* 2012, vol. 9, 791-802
- **CONNOLLY et al.** *Biophys. J.,* 2012, vol. 103, 69-78
- **BIRD et al.** *Science,* 1988, vol. 242, 42S-426
- **HUSTON et al.** *PNAS,* 1988, vol. 85, 5879-5883
- **ILL et al.** *Protein Eng,* 1997, vol. 10, 949-57
- **HOLLIGER ; HUDSON.** *Nat Biotechnol,* 2005, vol. 2S, 1126-1136
- Antibody Engineering, Methods in Molecular Biology. vol. 248
- **ANGAL et al.** *Mol Immunol.,* 1995, vol. 30, 105-8
- **KABAT et al.** Sequences of Proteins of Immunological Interest. NIH Publication No. 91-3242, 1991
- **CHOTHIA ; LESK.** *J. Mol. Biol,* 1987, vol. 196, 901-917
- Remington: The Science and Practice of Pharmacy. 1995
- **DAVENPORT et al.** *Internat. Immunopharmacol,* 2002, vol. 2, 653-672
- **HE et al.** *Anal Biochem,* 2010, vol. 399, 141-143